# EUROPÄISCHE PATENTSCHRIFT

(11) **EP 4 003 286 B1**
(45) Veröffentlichungstag und Bekanntmachung des Hinweises auf die Patenterteilung: **31.12.2025**
(21) Anmeldenummer: 20731454.3
(22) Anmeldetag: 08.06.2020
(51) Int. Cl.: A61K 8/58, A61K 8/25, A61K 8/33, A61K 8/35, A61Q 5/06, A61Q 5/08, A61Q 5/10, A61K 8/34

(54) **ERHÖHUNG DER STABILITÄT VON MITTELN ZUR BEHANDLUNG VON KERATINMATERIAL**
INCREASING THE STABILITY OF AGENTS FOR THE TREATMENT OF KERATIN MATERIAL
AUGMENTER LA STABILITÉ DES AGENTS POUR LE TRAITEMENT DES MATIÈRES KÉRATINIQUES

(30) Priorität: 24.07.2019 DE 102019210983
(43) Veröffentlichungstag der Anmeldung: 01.06.2022
(73) Patentinhaber: Henkel AG & Co. KGaA, 40589 Düsseldorf (DE)
(72) Erfinder: JAISER, Phillip, 40764 Langenfeld (DE); KRIENER, Caroline, 40229 Düsseldorf (DE); LECHNER, Torsten, 40764 Langenfeld (DE); WESER, Gabriele, 41472 Neuss (DE); NOWOTTNY, Marc, 41069 Mönchengladbach (DE); SCHOEPGENS, Juergen, 41366 Schwalmtal (DE); SCHUMACHER, Ulrike, 40589 Düsseldorf (DE); KOLONKO, Claudia, 42857 Remscheid (DE); MATHIASZYK, Carsten, 45277 Essen (DE); ERKENS, Udo, 47877 Willich (DE)
(86) Internationale Anmeldenummer: PCT/EP2020/065790
(87) Internationale Veröffentlichungsnummer: WO 2021/013420

(56) Entgegenhaltungen:
- WO-A1-2006/097158
- WO-A1-2018/115059
- WO-A1-2019/021487
- WO-A1-2019/214872
- WO-A1-2020/089353
- WO-A1-2020/089361
- DE-A1- 102005 039 456
- US-A1- 2010 083 446

## Beschreibung

Die vorliegende Anmeldung liegt auf dem Gebiet der Kosmetik und betrifft ein Verfahren zur Behandlung von keratinischem Material, insbesondere menschlichen Haaren, welches die Anwendung von drei Zusammensetzungen (A), (B) und (C) umfasst. Bei der Zusammensetzung (A) handelt es sich um eine wasserarme Zubereitung, die mindestens zwei organische C₁-C₆-Alkoxysilane enthält, und die Zusammensetzung (B) beinhaltet neben Wasser mindestens einen aromatischen oder aliphatischen Aldehyd mit 2 bis 20 Kohlenstoffatomen. Die Zusammensetzung (C) enthält mindestens eine farbgebende Verbindung aus der Gruppe der Pigmente und/oder der direktziehenden Farbstoffe.

Ein zweiter Gegenstand der vorliegenden Erfindung ist eine Mehrkomponenten-Verpackungseinheit (Kit-of-parts) zum Färben von keratinischem Material, welche getrennt konfektioniert in drei Verpackungseinheiten die drei zuvor beschriebenen Zusammensetzungen (A), (B) und (C) umfasst

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

EP 2168633 B1 und US 2010/0083446 A1 beschäftigen sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

Wenn diese Alkoxy-Silane bzw. ihre Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern und resultiert in überraschend waschbeständigen Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach sehr kurzen Anwendungszeiträumen von nur wenigen Minuten extrem gute Färbeergebnisse erzielt werden. Neben diesen Vorteilen birgt die hohe Reaktivität der Alkoxy-Silane jedoch auch einige Nachteile.

Aufgrund ihrer hohen Reaktivität können die organischen Alkoxy-Silane nicht zusammen mit größeren Wassermengen konfektioniert werden, da ein großer Überschuss an Wasser die sofortige Hydrolyse und im Anschluss daran eine Polymerisation initiiert. Die bei Lagerung der Alkoxy-Silane in wässrigem Medium stattfindende Polymerisierung äußert sich in einer Verdickung oder Gelierung der wässrigen Zubereitung. Hierdurch werden die Zubereitungen so hochviskos, gelig bzw. gallertartig, dass sie nicht mehr gleichmäßig auf dem Keratinmaterial aufgetragen werden können. Zudem ist die Lagerung der Alkoxy-Silane in Gegenwart hoher Wassermengen mit einem Verlust ihrer Reaktivität verbunden, so dass auch die Ausbildung eines widerstandsfähigen Coatings auf dem Keratinmaterial nicht mehr möglich ist.

Aus diesen Gründen ist es notwendig, die organischen Alkoxy-Silane in wasserfreiem bzw. wasserarmem Milieu zu lagern und die entsprechenden Zubereitungen in einem separaten Container zu konfektionieren. Aufgrund ihrer hohen Reaktivität können die Alkoxy-Silane nicht nur mit Wasser, sondern auch mit anderen kosmetischen Inhaltsstoffen reagieren. Zur Vermeidung aller unerwünschten Reaktionen enthalten die Zubereitungen mit Alkoxy-Silanen deshalb bevorzugt keine weiteren Inhaltsstoffe oder nur die ausgewählten Inhaltsstoffe, die sich gegenüber den Alkoxy-Silanen als chemisch inert herausgestellt haben. Die Konzentration der Alkoxy-Silane in der Zubereitung wird dementsprechend bevorzugt verhältnismäßig hoch gewählt. Die wasserarmen Zubereitungen, welche die Alkoxy-Silane in relativ hohen Konzentrationen enthalten, können auch als "Silan-Blend" bezeichnet werden.

Für die Applikation auf dem Keratinmaterial muss der Anwender nun diesen verhältnismäßig hoch konzentrierten Silan-Blend in eine anwendungsbereite Mischung überführen. In dieser anwendungsbereiten Mischung ist zum einen die Konzentration der organischen Alkoxy-Silane erniedrigt, und zum anderen enthält die Anwendungsmischung auch einen höheren Anteil an Wasser (bzw. einem alternativen Inhaltsstoff), durch welchen die zum Coating führende Polymerisation ausgelöst wird.

Es hat sich als extrem große Herausforderung herausgestellt, die Polymerisationsgeschwindigkeit, d.h. die Geschwindigkeit, mit der sich das Coating auf dem Keratinmaterial ausbildet, optimal an die Anwendungsbedingungen anzupassen.

Bei Anwendung auf menschlichen Haaren führt eine zu schnelle Polymerisationsgeschwindigkeit beispielsweise dazu, dass die Polymerisation bereits abgeschlossen ist, bevor alle Haarpartien behandelt werden konnten. Eine zu schnelle Polymerisation macht deshalb die Ganzkopfbehandlung unmöglich. Im Färbeprozess äußert sich die zu schnelle Polymerisation in einem extrem ungleichmäßigen Farbergebnis, so dass die Haarpartien, die zuletzt behandelt wurden, nur mangelhaft gefärbt sind.

Bei einer zu langsam ablaufenden Polymerisation können andererseits zwar alle Areale des Haares ohne Zeitdruck behandelt werden, jedoch steigt hierdurch die Anwendungsdauer bzw. die Einwirkzeit der Formulierungen auf das Keratinmaterial. Bei zu langsamer Polymerisation kommt daher der große Vorteil dieser Färbetechnologie, die Ausbildung von waschechten Färbungen innerhalb kürzester Anwendungszeiträume, nicht zum Tragen.

Es war die Aufgabe der vorliegenden Anmeldung, ein Verfahren zum Behandeln von keratinischem Material aufzufinden, mittels welchem die Polymerisationsgeschwindigkeit der organischen Alkoxy-silane an die Anwendungsbedingungen, insbesondere an die bei Anwendung auf dem menschlichen Kopf herrschenden Bedingungen, angepasst werden konnte. Mit anderen Worten wurde nach einem Verfahren gesucht, durch welches die organischen Alkoxy-silane so lange reaktiv bleiben, dass eine Ganzkopfbehandlung ermöglicht wird, ohne den Anwendungszeiträum über Gebühr zu verlängern.

Überraschenderweise hat sich herausgestellt, dass diese Aufgabe in vollem Umfang gelöst werden kann, wenn das Keratinmaterial in einem Verfahren behandelt wird, bei dem drei Zusammensetzungen (A), (B) und (C) auf dem Keratinmaterial angewendet werden. Bei der ersten Zusammensetzung (A) handelt es sich um den zuvor beschriebenen wasserarmen Silan-Blend. Die zweite Zusammensetzung (B) ist wasserhaltig und enthält zudem mindestens einen aromatischen oder aliphatischen Aldehyd mit 2 bis 20 Kohlenstoffatomen. Bei der Anwendung kommen beide Zusammensetzungen (A) und (B) miteinander in Kontakt, wobei dieser Kontakt entweder durch vorheriges Abmischen von (A) und (B) oder durch sukzessives Auftragen von (A) und (B) auf dem Keratinmaterial entstehen kann. Die dritte Zusammensetzung (C) enthält mindestens eine farbgebende Verbindung aus der Gruppe der Pigmente und/oder der direktziehenden Farbstoffe.

Ein erster Gegenstand der vorliegenden Erfindung ist ein Verfahren zum Behandeln von keratinischem Material, insbesondere menschlichen Haaren, bei dem auf dem keratinischen Material angewendet werden
- eine erste Zusammensetzung (A), die - bezogen auf das Gesamtgewicht der Zusammensetzung (A) - enthält
   (A1) weniger als 10 Gew.-% Wasser und
   (A21) mindestens ein organisches C₁-C₆-Alkoxy-Silan, das ausgewählt ist aus der Gruppe aus (3-Aminopropyl)triethoxysilan, (3-Aminopropyl)trimethoxysilan, (2-Aminoethyl)triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxysilan, (2-Dimethylaminoethyl)trimethoxysilanund/oder deren Kondensationsprodukten, und
   (A22) mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten, und
- eine zweite Zusammensetzung (B), die enthält
   (B1) Wasser und
   (B2) einen oder mehrere aromatische oder aliphatische Aldehyde mit 2 bis 20 Kohlenstoffatomen, und
- eine dritte Zusammensetzung (C), die enthält
   (C1) mindestens eine farbgebende Verbindung aus der Gruppe der Pigmente und/oder der direktziehenden Farbstoffe.

Es hat sich gezeigt, dass die in der wasserhaltigen Zusammensetzung (B) enthaltenen Aldehyde (B2) bei Kontakt mit der Zusammensetzung (A) die Polymerisationsgeschwindigkeit der organischen C₁-C₆-Alkoxy-Silane (A21) und (A22) reduzieren. Überraschenderweise konnte die Reaktivität der organische C₁-C₆-Alkoxy-Silane (A21) und (A22) so optimal an die bei einem Ganzkopf-Haarfärbeprozess herrschenden Anwendungsbedingungen angepasst werden. Auch kompliziertere bzw. zeitaufwändigere Färbetechniken wie beispielsweise die Färbung von speziell auf dem Kopf angeordneten Strähnchen, konnten bei Anwendung des erfindungsgemäßen Verfahrens realisiert werden. Bei Einsatz der beiden Zusammensetzungen (A) und (B) in einem Färbeverfahren auf Keratinmaterial, insbesondere auf menschlichen Haaren, konnten auf diese Weise Färbungen mit besonders hoher Gleichmäßigkeit, Reibechtheit und Waschechtheit erzeugt werden.

### Behandlung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter Mitteln zur Behandlung von keratinischem Material werden beispielsweise Mittel zur Färbung des Keratinmaterials, Mittel zur Umformung oder Formgebung von keratinischem Material, insbesondere keratinischen Fasern, oder auch Mittel zur Konditionierung bzw. zur Pflege des keratinischen Materials verstanden. Besonders gute Eignung zeige die über das erfindungsgemäße Verfahren hergestellten Mittel zur Färbung von keratinischem Material, insbesondere zur Färbung von keratinischen Fasern, bei denen es sich besonders bevorzugt um menschliche Haare handelt.

Der Begriff "Mittel zur Färbung" wird im Rahmen dieser Erfindung für eine durch Einsatz von farbgebenden Verbindungen, wie beispielsweise Pigmenten, Mica, direktziehenden Farbstoffen, thermochromen und photochromen Farbstoffen hervorgerufene Farbgebung des Keratinmaterials, insbesondere des Haares, verwendet. Ganz besonders bevorzugt ist der Einsatz von Pigmenten. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Polymerisierung der organischen Alkoxy-Silane, und durch die Wechselwirkung von farbgebender Verbindung und organischen Siliciumverbindungen und optional weiteren Bestandteilen, wie beispielsweise einem filmbildenden, Polymer.

### Wassergehalt (A1) in der Zusammensetzung (A)

Das erfindungsgemäße Verfahren ist gekennzeichnet durch die Anwendung einer ersten Zusammensetzung (A) auf dem keratinischen Material.

Zur Gewährleistung einer ausreichend hohen Lagerstabilität ist die Zusammensetzung (A) dadurch gekennzeichnet, dass sie wasserarm, bevorzugt im wesentlichen wasserfrei, ist. Deshalb enthält die Zusammensetzung (A) - bezogen auf das Gesamtgewicht der Zusammensetzung (A) - weniger als 10 Gew.-% Wasser.

Bei einem Wassergehalt von knapp unter 10 Gew.-% sind die Zusammensetzungen (A) über längere Zeiträume lagerstabil. Um die Lagerstabilität noch weiter zu verbessern und um eine ausreichend hohe Reaktivität der organischen C₁-C₆-Alkoxy-Silane (A21) und (A22) zu gewährleisten, hat es sich jedoch als besonders bevorzugt herausgestellt, den Wassergehalt in der Zusammensetzung (A) noch weiter herabzusenken. Aus diesem Grund enthält erste Zusammensetzung (A) - bezogen auf das Gesamtgewicht der Zusammensetzung (A) - bevorzugt 0,01 bis 9,5 Gew.-%, weiter bevorzugt 0,01 bis 8,0 Gew.-%, noch weiter bevorzugt 0,01 bis 6,0 und ganz besonders bevorzugt 0,01 bis 4,0 Gew.-% Wasser (A1).

Im Rahmen einer ganz besonders bevorzugten Ausführungsform ist ein erfindungsgemäßes Verfahren dadurch gekennzeichnet, dass die erste Zusammensetzung (A) - bezogen auf das Gesamtgewicht der Zusammensetzung (A) - 0,01 bis 9,5 Gew.-%, bevorzugt 0,01 bis 8,0 Gew.-%, weiter bevorzugt 0,01 bis 6,0 und ganz besonders bevorzugt 0,01 bis 4,0 Gew.-% Wasser (A1) enthält.

### Organische C₁-C₆-Alkoxy-Silane (A21) und (A22) und/oder deren Kondensationsprodukte in der Zusammensetzung (A)

Die Zusammensetzung (A) ist dadurch gekennzeichnet, dass sie ein oder mehrere organische C₁-C₆-Alkoxy-Silane (A21) und/oder deren Kondensationsprodukte und ein oder mehrere organische C₁-C₆-Alkoxy-Silane (A22) und/oder deren Kondensationsprodukte enthält.

Bei den organischen C₁-C₆-Alkoxy-Silanen handelt es sich um organische, nicht polymere Siliciumverbindungen,

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind bevorzugt Verbindungen, die ein bis drei Silicumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt.

Kennzeichnend für die erfindungsgemäßen C₁-C₆-Alkoxy-Silane ist, dass mindestens eine C₁-C₆-Alkoxygruppe direkt an ein Siliciumatom gebunden vorliegt. Die erfindungsgemäßen C₁-C₆-Alkoxy-Silane umfassen damit mindestens eine Struktureinheit R'R"R‴Si-O-(C₁-C₆-Alkyl) wobei die Reste R', R" und R‴ für die drei übrigen Bindungsvalenzen des Siliciumatoms stehen.

Das oder diese an das Siliciumatom gebundenen C₁-C₆-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren C₁-C₆-Alkoxy-Gruppe um eine Ethoxygruppe, so enthält die organische Siliciumverbindung bevorzugt eine Struktureinheit R'R"R‴Si-O-CH2-CH3. Die Reste R', R" und R‴ stellen wieder die drei übrigen freien Valenzen des Siliciumatoms dar.

Bereits der Zusatz geringer Wassermengen führt zunächst zur Hydrolyse und dann zu einer Kondensationsreaktion der organischen Alkoxy-silane untereinander. Aus diesem Grund können sowohl die organischen Alkoxy-silane (A21) bwz. (A22) als auch deren Kondensationsprodukte in der Zusammensetzung enthalten sein.

Unter einem Kondensationsprodukt wird ein Produkt verstanden, dass durch Reaktion von mindestens zwei organischen C₁-C₆-Alkoxy-Silanen unter Abspaltung von Wasser und/oder unter Abspaltung von einem C₁-C₆-Alkanol entsteht.

Die Kondensationsprodukte können beispielsweise Dimere, aber auch Trimere oder Oligomere sein, wobei die Kondensationsprodukte mit den Monomeren im Gleichgewicht stehen.

Abhängig von der eingesetzten bzw. in der Hydrolyse verbrauchten Wassermenge verschiebt sich das Gleichgewicht von monomerem C₁-C₆-Alkoxysilan zu Kondensationsprodukt.

Zur Lösung der erfindungsgemäßen Aufgabenstellung besonders gut geeignete organische Silicumverbindungen (A21) sind
- (3-Aminopropyl)triethoxysilan 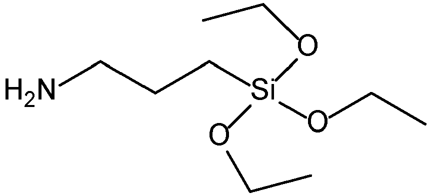
- (3-Aminopropyl)trimethoxysilan 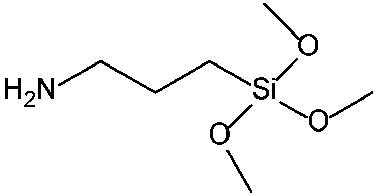
- (2-Aminoethyl)triethoxysilan 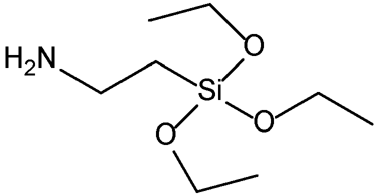
- (2-Aminoethyl)trimethoxysilan 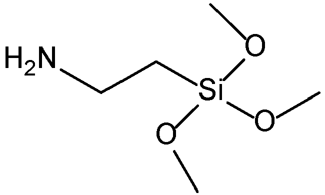
- (3-Dimethylaminopropyl)triethoxysilan 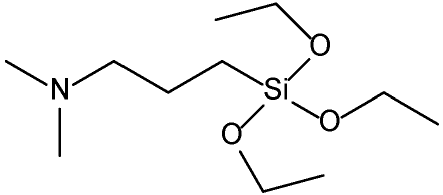
- (3-Dimethylaminopropyl)trimethoxysilan 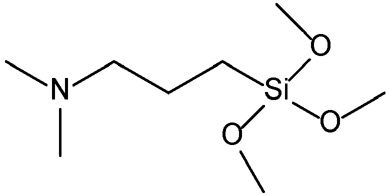
- (2-Dimethylaminoethyl)triethoxysilan. 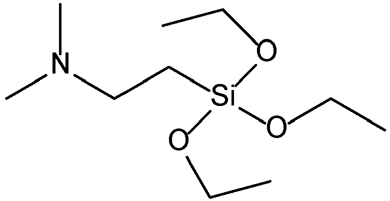
- (2-Dimethylaminoethyl)trimethoxysilan und/oder

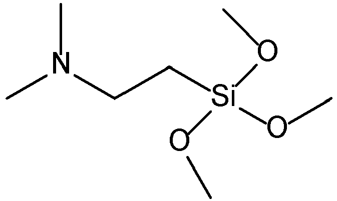

Das erfindungsgemäße Verfahren ist dadurch gekennzeichnet, dass die erste Zusammensetzung (A) mindestens ein organisches C₁-C₆-Alkoxysilan (A21) enthält, das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten.

Die vorgenannten organische Siliciumverbindungen (A21) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

In weiteren Färbeversuchen hat es sich ebenfalls als ganz besonders vorteilhaft herausgestellt, wenn im erfindungsgemäßen Verfahren mindestens ein organisches C₁-C₆-Alkoxy-Silan (A22) eingesetzt wurde

Zur Lösung der erfindungsgemäßen Aufgabenstellung besonders gut geeignete organische Silicumverbindungen (A22) sind
- Methyltrimethoxysilan 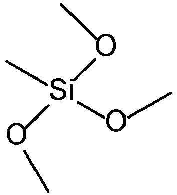
- Methyltriethoxysilan 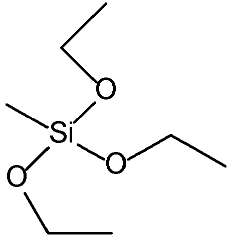
- Ethyltrimethoxysilan 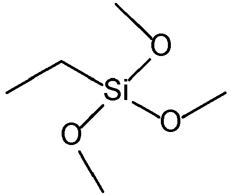
- Ethyltriethoxysilan 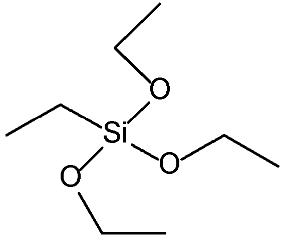
- n-Hexyltrimethoxysilan (auch bezeichnet als Hexyltrimethoxysilan) 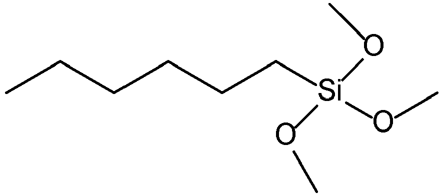
- n-Hexyltriethoxysilan (auch bezeichnet als Hexyltriethoxysilan) 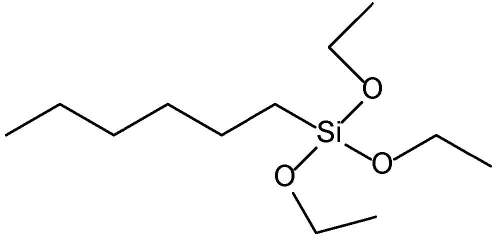
- n-Octyltrimethoxysilan (auch bezeichnet als Octyltrimethoxysilan) 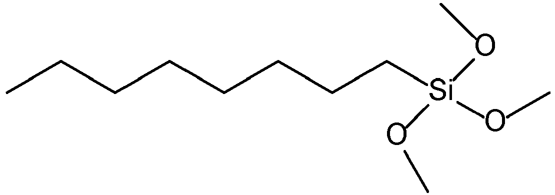
- n-Octyltriethoxysilan (auch bezeichnet als Octyltriethoxysilan) 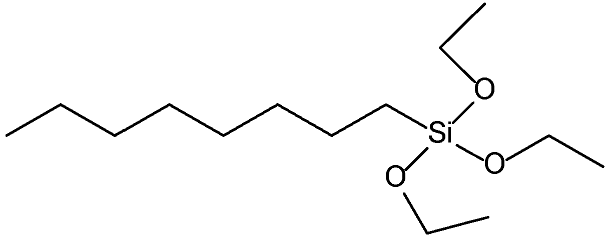
- n-Dodecyltrimethoxysilan (auch bezeichnet als Dodecyltrimethoxysilan) und/oder 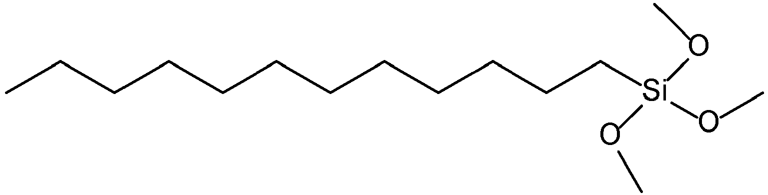
- n-Dodecyltriethoxysilan (auch bezeichnet als Dodecyltriethoxysilan).

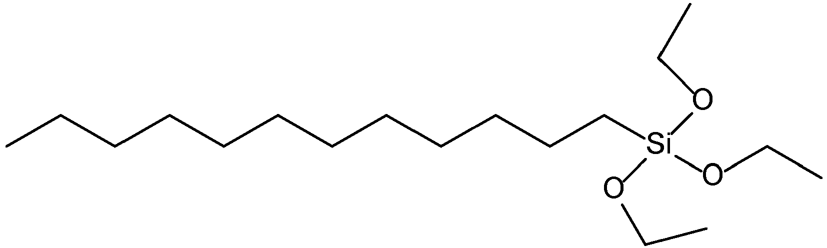

Das erfindungsgemäße Verfahren ist dadurch gekennzeichnet, dass die erste Zusammensetzung (A) mindestens ein organisches C₁-C₆-Alkoxysilan (A22) enthält, das ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
und/oder deren Kondensationsprodukten.

Bei den entsprechenden Hydrolyse bzw. Kondensationsprodukten handelt es sich beispielsweise um die folgenden Verbindungen:
Hydrolyse von C₁-C₆-Alkoxy-Silan der Formel (S-I) mit Wasser (Reaktionsschema am Beispiel von 3-Aminopropyltriethoxysilan):

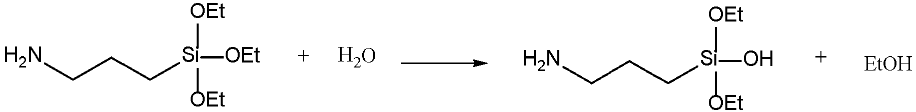

In Abhängigkeit von der eingesetzten Menge an Wasser kann die Hydrolyse-Reaktion auch mehrfach pro eingesetztem C₁-C₆-Alkoxy-Silan stattfinden: 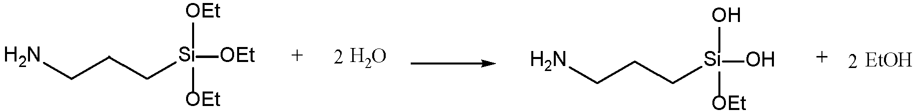 bzw.

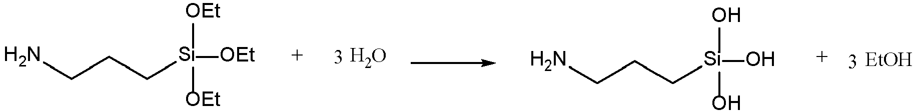

Hydrolyse von C₁-C₆-Alkoxy-Silan der Formel (S-IV) mit Wasser (Reaktionsschema am Beispiel von Methyltrimethoxysilan):

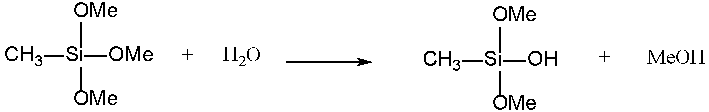

In Abhängigkeit von der eingesetzten Menge an Wasser kann die Hydrolyse-Reaktion auch mehrfach pro eingesetztem C₁-C₆-Alkoxy-Silan stattfinden: 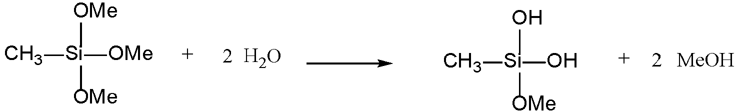 bzw.

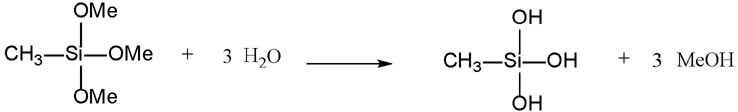

Mögliche Kondensationsreaktionen sind beispielsweise (gezeigt anhand des Gemisches (3-Aminopropyl)triethoxysilan und Methyltrimethoxysilan): 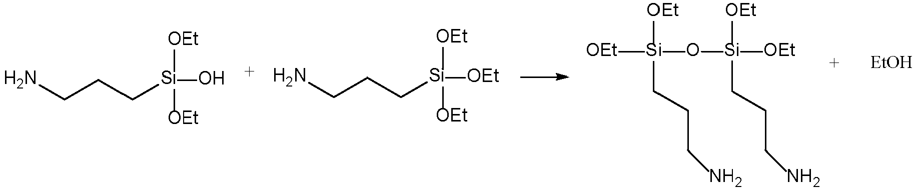 und/oder 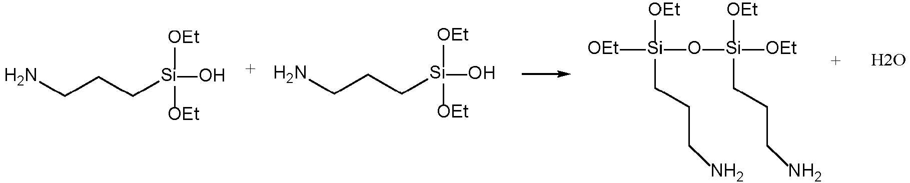 und/oder 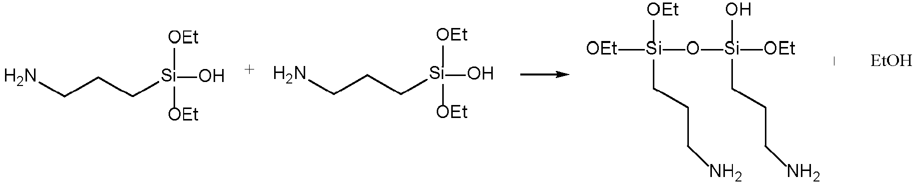 und/oder 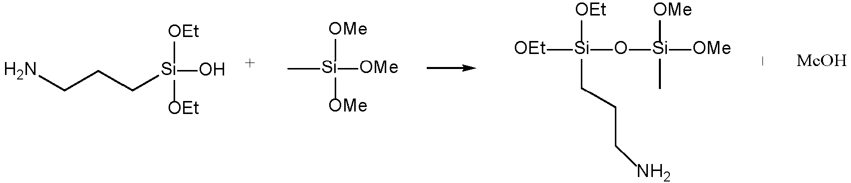 und/oder 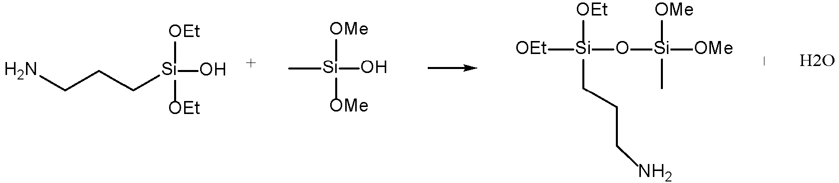 und/oder 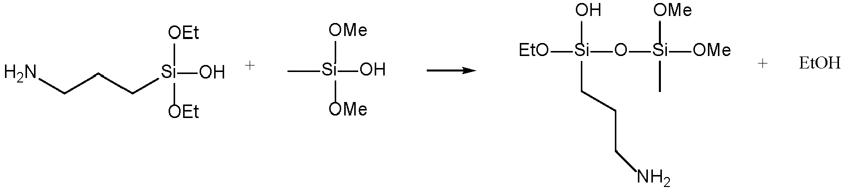 und/oder

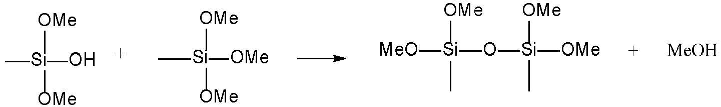

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt.

An diesen Kondensationsreaktionen können sowohl partiell hydrolysierte als auch vollständig hydrolysierte C₁-C₆-Alkoxysilane der Formel (S-I) teilnehmen, die eine Kondensation mit noch nicht abreagierten, partiell oder auch vollständig hydrolysierten C₁-C₆-Alkoxysilanen der Formel (S-I) durchlaufen. In diesem Fall reagieren die C₁-C₆-Alkoxysilane der Formel (S-I) mit sich selbst.

Weiterhin können an den Kondensationsreaktionen auch sowohl partiell hydrolysierte als auch vollständig hydrolysierte C₁-C₆-Alkoxysilane der Formel (S-I) teilnehmen, die eine Kondensation mit noch nicht abreagierten, partiell oder auch vollständig hydrolysierten C₁-C₆-Alkoxysilanen der Formel (S-IV) durchlaufen. In diesem Fall reagieren die C₁-C₆-Alkoxysilane der Formel (S-I) mit den C₁-C₆-Alkoxysilanen der Formel (S-IV).

Weiterhin können an den Kondensationsreaktionen auch sowohl partiell hydrolysierte als auch vollständig hydrolysierte C₁-C₆-Alkoxysilane der Formel (S-IV) teilnehmen, die eine Kondensation mit noch nicht abreagierten, partiell oder auch vollständig hydrolysierten C₁-C₆-Alkoxysilanen der Formel (S-IV) durchlaufen. In diesem Fall reagieren die C₁-C₆-Alkoxysilane der Formel (S-IV) mit sich selbst.

Die erfindungsgemäße Zusammensetzung (A) kann ein oder mehrere organische C₁-C₆-Alkoxy-silane (A2) in verschiedenen Mengenanteilen enthalten. Diese bestimmt der Fachmann in Abhängigkeit von der gewünschten Dicke des Silan-Coatings auf dem Keratinmaterial und von der Menge des zu behandelnden Keratinmaterials.

Besonders lagerstabile Zubereitungen mit sehr gutem Färberesultat bei der Anwendung konnten erhalten werden, wenn die Zusammensetzung (A) - bezogen auf ihr Gesamtgewicht - ein oder mehrere organische C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

In einer weiteren Ausführungsform ist ein ganz besonders bevorzugtes Verfahren dadurch gekennzeichnet, dass die erste Zusammensetzung (A) - bezogen auf das Gesamtgewicht der Zusammensetzung (A) - ein oder mehrere organische C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

### Weitere kosmetische Inhaltsstoffe in der Zusammensetzung (A)

Prinzipiell kann die Zusammensetzung (A) auch noch einen oder mehrere weitere kosmetische Inhaltsstoffe enthalten.

Bei den fakultativ in der Zusammensetzung (A) einsetzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Wie bereits zuvor beschrieben, können die organischen C₁-C₆-Alkoxysilane (A21) und (A22) jedoch nicht nur mit Wasser, sondern auch mit anderen kosmetischen Inhaltsstoffen reagieren. Zur Vermeidung dieser unerwünschten Reaktionen enthalten die Zubereitungen (A) mit Alkoxy-Silanen deshalb bevorzugt keine weiteren Inhaltsstoffe oder nur die ausgewählten Inhaltsstoffe, die sich gegenüber den C₁-C₆-Alkoxy-Silanen als chemisch inert herausgestellt haben. Als ganz besonders bevorzugt hat es sich in diesem Zusammenhang erwiesen, in der Zusammensetzung (A) einen kosmetischen Inhaltsstoff aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan einzusetzen.

In einer weiteren ganz besonders bevorzugten Ausführungsform ist ein erfindungsgemäßes Verfahren dadurch gekennzeichnet, das die erste Zusammensetzung (A) zusätzlich mindestens einen kosmetischen Inhaltsstoff aus der Gruppe aus Hexamethyldisiloxan. Octamethyltrisiloxan, Decamethyltetrasiloxan, Hexamethylcyclotrisiloxan, Octamethylcyclotetrasiloxan und Decamethylcyclopentasiloxan enthält.

Hexamethyldisiloxan besitzt die CAS-Nummer 107-46-0 und kann beispielsweise bei Sigma-Aldrich kommerziell erworben werden.

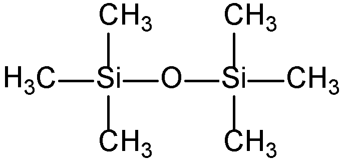

Octamethyltrisiloxan besitzt die CAS-Nummer 107-51-7 und ist ebenfalls bei Sigma-Aldrich kommerziell erhältlich.

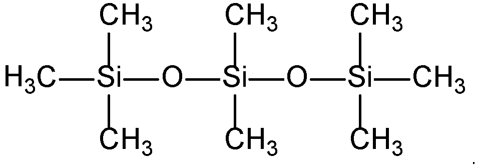

Decamethyltetrasiloxan trägt die CAS-Nummer 141-62-8 und ist ebenfalls bei Sigma-Aldrich kommerziell erhältlich. 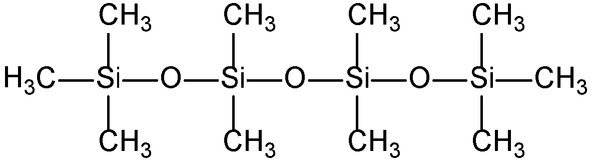
Hexamethylcyclotrisiloxan besitzt die CAS-Nr. 541-05-9.
Octamethylcyclotetrasiloxan besitzt die CAS-Nr. 556-67-2.
Decamethylcyclopentasiloxan besitzt die CAS-Nr. 541-02-6.

Als ganz besonders bevorzugt hat sich der Einsatz von Hexamethyldisiloxan in der Zusammensetzung (A) herausgestellt. Besonders bevorzugt ist Hexamethyldisiloxan - bezogen auf das Gesamtgewicht der Zusammensetzung (A) - in Mengen von 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% in der Zusammensetzung (A) enthalten.

In einer weiteren ganz besonders bevorzugten Ausführungsform ist ein erfindungsgemäßes Verfahren dadurch gekennzeichnet, dass die erste Zusammensetzung (A) - bezogen auf das Gesamtgewicht der Zusammensetzung (A) - 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan enthält.

### Wassergehalt (B1) in der Zusammensetzung (B)

Kennzeichnend für das erfindungsgemäße Verfahren ist die Anwendung einer zweiten Zusammensetzung (B) auf dem keratinischen Material, insbesondere auf den menschlichen Haaren.

Bei der Anwendung auf dem keratinischen Material kommen die Zusammensetzungen (A) und (B) in Kontakt, wobei dieser Kontakt ganz besonders bevorzugt durch vorheriges Vermischen der beiden Zusammensetzungen (A) und (B) hergestellt werden kann. Durch das Vermischen von (A) und (B) wird das anwendungsbereite Keratinbehandlungsmittel hergestellt, d.h. der lagerstabile bzw. lagerfähige Silan-Blend (A) wird durch Kontakt mit (B) in seine reaktive Form überführt. Mit Vermischen der Zusammensetzungen (A) und (B) startet eine von den Alkoxy-Silan-Monomeren oder Alkoxy-Silan-Oligomeren ausgehende Polymerisationsreaktion, die schließlich zur Ausbildung des Films bzw. des Coatings auf dem Keratinmaterial führt.

Je mehr Wasser mit dem oder den organischen C₁-C₆-Alkoxy-Silanen in Kontakt kommt, in desto stärkerem Ausmaß läuft die Polymerisationsreaktion ab. Enthält die Zusammensetzung (B) beispielsweise sehr viel Wasser, so polymerisieren die zuvor in der wasserarmen Zusammensetzung (A) vorhandenen monomeren bzw. oligomeren Silan-Kondensate nun sehr schnell zu Polymeren höheren oder hohen Molekulargewichts. Die hochmolekularen Silan-Polymere bilden dann den Film auf dem keratinischen Material. Aus diesem Grund ist Wasser (B1) ein erfindungswesentlicher Inhaltsstoff der Zusammensetzung (B).

Durch die Menge an Wasser in der Zusammensetzung (B) kann die Polymerisationsgeschwindigkeit der organischen C₁-C₆-Alkoxy-Silane (A21) und (A22) zum Zeitpunkt der Anwendung mitbestimmt werden. Um bei einer Haarfärbung auf dem gesamten Kopf ein gleichmäßiges Farbergebnis zu gewährleisten, sollte die Polymerisationsgeschwindigkeit, d.h. die Schnelligkeit, mit der sich das Coating ausbildet, jedoch auch nicht zu hoch sein. Aus diesem Grund hat es sich als ganz besonders bevorzugt herausgestellt, die Menge an Wasser in der Zusammensetzung (B) nicht zu hoch zu wählen.

Besonders gleichmäßige Färbungen auf dem gesamten Kopf konnten erhalten werden, wenn die Zusammensetzung (B) - bezogen auf das Gesamtgewicht der Zusammensetzung (B) - 5,0 bis 90,0 Gew.-%, bevorzugt 15,0 bis 85,0 Gew.-%, weiter bevorzugt 25,0 bis 80,0 Gew.-% , nocht weiter bevorzugt 35,0 bis 75,0 Gew.-% und ganz besonders bevorzugt 45,0 bis 70,0 Gew.-% Wasser (B1) enthält.

Im Rahmen einer weiteren ganz besonders bevorzugten Ausführungsform ist ein erfindungsgemäßes Verfahren dadurch gekennzeichnet dass die zweite Zuammensetzung (B) - bezogen auf das Gesamtgewicht der Zusammensetzung (B) - 5,0 bis 90,0 Gew.-%, bevorzugt 15,0 bis 85,0 Gew.-%, weiter bevorzugt 25,0 bis 80,0 Gew.-% , nocht weiter bevorzugt 35,0 bis 75,0 Gew.-% und ganz besonders bevorzugt 45,0 bis 70,0 Gew.-% Wasser (B1) enthält.

### Aldehyde (B2) in der Zusammensetzung (B)

Kennzeichnend für die Zusammensetzung (B) ist weiterhin ihr Gehalt an mindestens einem aromatischen oder aliphatischen Aldehyd mit 2 bis 20 Kohlenstoffatomen (B2).

Unter Aldehyden werden organische Verbindungen verstanden, die als funktionelle Gruppe mindestens eine Aldehyd-Gruppe -CHO besitzen. Erfindungsgemäße Aldehyde (B2) können auch zwei oder mehr Aldehyd-Gruppen besitzen. Zusätzlich zu dieser mindestens einen Aldehyd-Gruppe kann die organische Verbindung auch noch weitere funkionelle Gruppen tragen, wie beipsielsweise mindestens eine Hydroxygruppe, mindestens eine C₁-C₆-Alkylgruppe, mindestens eine C₁-C₆-Alkoxygruppe, mindestens ein Halogenatom aus der Gruppe aus Fluor, Chlor und Brom, mindestens eine Aminogruppe, mindestens eine Di-C₁-C₆-Alkylaminogruppe, mindestens eine Carboxygruppe - COOH oder ein Salz hiervon oder mindestens eine Nitrogruppe.

Erfindungsgemäße Aldehyde sind aus 2 bis 20 Kohlenstoffatome aufgebaut und können aromatisch oder aliphatisch sein.

Ein aromatischer Aldehyd umfasst mindestens einen aromatischen Ring, der 5-gliedrig oder ganz besonders bevorzugt 6-gliedrig sein kann. Aromatische 5-Ringe sind bevorzugt heterozyklisch. Aromatische 6-Ringe können heterozyklisch oder carbozyklisch sein.

Dementsprechend umfassen aromatische , carbozyklische Aldehyde in der Regel mindestens 7 Kohlenstoffatome (aromatischer, carbozyklischer 6-Ring plus mindestens eine Aldehydgruppe) und höchstens 20 Kohlenstoffatome. Als aromatischen carbozyklischen Ring können die erfindungsgemäßen Aldehyde zum Beispiel einen Benzen-Ring oder einen Naphthalen-Ring umfassen.

Aliphatische Aldehyde sind Verbindungen, die kein aromatisches Ringsystem enthalten. Aliphatische Verbindungen können auf Alkylgruppen oder Alkylketten basieren, wobei die Ketten von Heteroatomen unterbrochen sein können, und wobei die gesamte Kette unverzweigt oder verzeigt sein kann. Ebenso kann es sich bei aliphatischen Verbindungen auch um cyclische Verbindungen handeln, die aber kein aromatisches Ringsystem ausbilden. Als carbocyclischer, nicht aromatischer Ring kann beispielsweise ein Cycloalkanring, wie z.B. ein Cyclohexan- oder ein Cyclopentanring, genannt werden.

Überraschenderweise hat sich herausgestellt, dass der Einsatz mindestens eines aromatischen oder aliphatischen Aldehyds mit 2 bis 20 Kohlenstoffatomen (B2) die Reaktionsgeschwindigkeit der organischen C₁-C₆-Alkoxy-silane so optimiert, dass eine besonders gleichmäßige Färbung auf dem gesamten Kopf ermöglicht wird.

Prinzipiell kann diese Optimierung der Reaktionsgeschwindigkeit sowohl mit aromatischen als auch mit aliphatischen Aldehyden erzielt werden. Die besten Effekte wurden jedoch beobachet, wenn in der zweiten Zusammensetzung (B) mindestens einen aromatischen, carbozyklischen Aldehyd (B2) mit 7 bis 20 Kohlenstoffatomen enthält.

Im Rahmen einer weiteren ganz besonders bevorzugten Ausführungsform ist ein erfindungsgemäßes Verfahren dadurch gekennzeichnet, dass die zweite Zusammensetzung (B) mindestens einen aromatischen, carbozyklischen Aldehyd (B2) mit 7 bis 20 Kohlenstoffatomen enthält.

Als Carbozyklen werden cyclische Verbindungen bezeichnet, die ausschließlich Kohlenstoffatome im Ring enthalten. Ein erfindungsgemäßer aromatischen, carbozyklischen Aldehyd (B2) mit 7 bis 20 Kohlenstoffatomen besitzt demnach einen aromatischen Ring, wobei das Ringsystem selbst ausschließlich aus Kohlenstoffatomen aufgebaut wird.

Der einfachste aromatische carbozyklische Aldehyde (B2) ist Benzaldehyd, wobei der aromatische Ring jedoch besonders bevorzugt noch weitere Substituenten tragen kann.

Zur Lösung der erfindungsgemäßen Aufgabenstellung ganz besonders gut geeignete aromatische, carbozyklische Aldehyde (B2) sind Verbindungen der allgemeinen Formel (A-I) 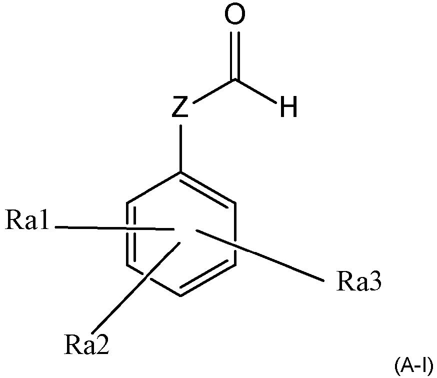 wobei
- Ra1, Ra2 und Ra3 stehen unabhängig voneinander für ein Wasserstoffatom, eine Hydroxygruppe, eine C₁-C₆-Alkoxygruppe, eine C₁-C₆-Alkylgruppe, ein Halogenatom, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe, oder aber
- Ra1 und Ra2 können zusammen mit den Kohlenstoffatomen des Benzenringes, an den sie gebunden sind, einen gesättigten oder ungesättigten, 5-gliedrigen oder 6-gliedrigen heterozyklischen oder carbozyklischen Ring ausbilden, und
- Z steht für eine direkte Bindung oder eine Vinylengruppe.

Im Rahmen einer weiteren ganz besonders bevorzugten Ausführungsform ist ein erfindungsgemäßes Verfahren dadurch gekennzeichnet, dass die zweite Zusammensetzung (B) mindestens einen aromatischen, carbozyklischen Aldehyd (B2) der allgemeinen Formel (A-I) enthält, 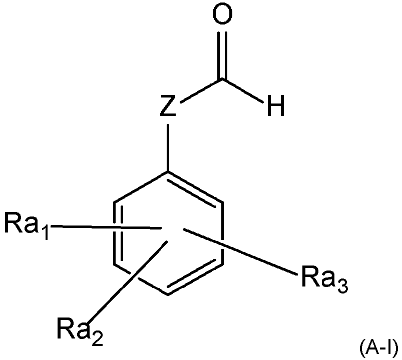 wobei
- Ra1, Ra2, Ra3: stehen unabhängig voneinander für ein Wasserstoffatom, eine Hydroxygruppe, eine C₁-C₆-Alkoxygruppe, eine C₁-C₆-Alkylgruppe, ein Halogenatom, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe, oder aber
- Ra1 und Ra2: können zusammen mit den Kohlenstoffatomen des Benzenringes, an den sie gebunden sind, einen gesättigten oder ungesättigten, 5-gliedrigen oder 6-gliedrigen heterozyklischen oder carbozyklischen Ring ausbilden, und
- Z: steht für eine direkte Bindung oder eine Vinylengruppe.

In diesem Zusammenhang erfolgt die Auswahl der Reste Ra1, Ra2, Ra3 und Z so, dass der resultierende Aldehyd zwischen 7 und 20 Kohlenstoffatomen besitzt.

Wurden ganz bestimmte Aldehyde (B2) der allgemeinen Formel (A-I) eingesetzt, so waren diese ganz besonders gut in der Lage, die die Polymerisationsgeschwindigkeit der organischen C₁-C₆-Alkoxy-Silane (A21) und (A22) reduzieren. Aus diesem Grund wurde bei Verwendung dieser ganz besonders bevorzugten Aldehyde (B2) Färbungen erhalten, die sich über eine ganz besonders hohe Farbintensität, Gleichmäßigkeit, Reibechtheit und Waschechheit auszeichneten.

Ganz besonders bevorzugte Aldehyde der allgemeinen Formel (A-I) können ausgewählt werden aus der Gruppe, bestehend aus Benzaldehyd und seinen Derivaten, Naphthaldehyd und seinen Derivaten, Zimtaldehyd und seinen Derivaten.

Ganz besonders bevorzugte Aldehyde der allgemeinen Formel (A-I) können ausgewählt werden aus der Gruppe, bestehend aus 4-Hydroxy-3-methoxybenzaldehyd, 4-Hydroxy-3-ethoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxybenzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 3,5-Dichlor-4-hydroxybenzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd und 4-Diphenylamino-benzaldehyd.

Im Rahmen einer weiteren ganz besonders bevorzugten Ausführungsform ist ein erfindungsgemäßes Verfahren dadurch gekennzeichnet, dass die zweite Zusammensetzung (B) mindestens einen aromatischen, carbozyklischen Aldehyd (B2) enthält, der ausgewählt ist aus der Gruppe aus 4-Hydroxy-3-methoxybenzaldehyd, 4-Hydroxy-3-ethoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxybenzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxybenzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 3,5-Dichlor-4-hydroxybenzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd und 4-Diphenylamino-benzaldehyd.

Durch Wahl der geeigneten Einsatzmengen an Aldehyden (B2) kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere Aldehyde (B2) in ganz bestimmten Mengenbereichen einzusetzen.

Es ist ganz besonders bevorzugt, wenn die zweite Zusammensetzung (B) - bezogen auf das Gesamtgewicht der Zusammensetzung (B) - ein oder mehrere aromatische oder aliphatischen Aldehyde mit 2 bis 20 Kohlenstoffatomen (B2) in einer Gesamtmenge von 0,1 bis 50,0 Gew.-%, bevorzugt 0,5 bis 10,0 Gew.-%, weiter bevorzugt von 0,7 bis 7,0 Gew.-% und ganz besonders bevorzugt von 1,0 bis 4,0 Gew.-% enthält.

Im Rahmen einer weiteren ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - ein oder mehrere aromatische oder aliphatischen Aldehyde mit 2 bis 20 Kohlenstoffatomen (B2) in einer Gesamtmenge von 0,1 bis 50,0 Gew.-%, bevorzugt 0,5 bis 10,0 Gew.-%, weiter bevorzugt von 0,7 bis 7,0 Gew.-% und ganz besonders bevorzugt von 1,0 bis 4,0 Gew.-% enthält.

Insbesondere ist es ganz besonders bevorzugt, wenn die zweite Zusammensetzung (B) - bezogen auf das Gesamtgewicht der Zusammensetzung (B) - ein oder mehrere Aldehyde (B2) der allgemeinen Formel (A-I) in einer Gesamtmenge von 0,1 bis 50,0 Gew.-%, bevorzugt 0,5 bis 10,0 Gew.-%, weiter bevorzugt von 0,7 bis 7,0 Gew.-% und ganz besonders bevorzugt von 1,0 bis 4,0 Gew.-% enthält.

Die allerbesten Ergebnisse im Hinblick auf die Farbintensität, die Waschechtheit und die Reibechtheit der mit dem erfindungsgemäßen Verfahren erzielbaren Färbungen wurden erhalten, wenn die Zusammensetzung (B) Vanillin (B2) enthielt. Der Einsatz von Vanillin als Aldehyd (B2) ist daher am allermeisten bevorzugt.

Im Rahmen einer weiteren ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) 0,1 bis 50,0 Gew.-%, bevorzugt 0,5 bis 10,0 Gew.-%, weiter bevorzugt von 0,7 bis 7,0 Gew.-% und ganz besonders bevorzugt von 1,0 bis 4,0 Gew.-% Vanillin (B2) enthält.

Vanillin ist wie auch die anderen genannten Aldehyde bei den gängigem, dem Fachmann bekannten Lieferanten von Chemikalien, wie beispeilsweise Sigma-Aldrich, Fluka oder Merck, kommerziell zu erwerben. So kann beispielsweise Vanillin mit der CAS-Nummer 121-33-5 in verschiedenen Gebindegrößen von Sigma-Aldrich kommerziell bezogen werden.

### Fettbestandteile in der Zusammensetzung (B)

Zur Einstellung der Viskosität bzw. zur weiteren Verbesserung der anwendungstechnischen Eigenschaften kann die Zusammensetzung (B) optional zusätzlich auch mindestens einen Fettbestandteil enthalten.

Bei den Fettbestandteilen handelt es sich um hydrophobe Substanzen, die in Gegenwart von Wasser unter Ausbildung von Mizell-Systemen Emulsionen formen können. Ohne auf diese Theorie festgelegt zu sein, wird vermutet, dass die C₁-C₆-Alkoxysilane - entweder in Form ihrer Monomere oder gegebenenfalls in Form ihrer kondensierten Oligomere - in diese hydrophobe Umgebung bzw. in die Mizell-Systeme eingebettet werden, so dass sich die Polarität ihrer Umgebung verändert. Bedingt durch den hydrophoben Charakter der Fettbestandteile wird auch die Umgebung der C₁-C₆-Alkoxysilane hydrophobiert. Es wird angenommen, dass die zum Film bzw. Coating führende Polymerisationsreaktion der C₁-C₆-Alkoxy-silane in einem Milieu verringerter Polarität mit reduzierter Geschwindigkeit abläuft.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen.

Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt werden die in der Zusammensetzung (B) enthaltenen Fettbestandteile (B2) ausgewählt aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe.

Als ganz besonders bevorzugte Fettbestandteile werden in diesem Zusammenhang die Bestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den C₁₂-C₃₀-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13*Z*)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Durch Auswahl besonders gut geeigneter Fettbestanteile kann die Polarität der Zusammensetzung (B) optimal eingestellt werden und die Polymerisationsgeschwindigkeit der C₁-C₆-Alkoxysilane besonders gut an die jeweils gewählten Anwendungsbedingungen angepasst werden.

In diesem Zusammenhang hat sich herausgestellt, dass insbesondere der Einsatz mindestens eines C₁₂-C₃₀-Fettalkohols (B2) in der Zusammensetzung (B) ein Emulsions-System schafft, in das die Alkoxysilane (A21) und (A22) besonders gut eingebettet werden können.

Im Rahmen einer Ausführungsform wurden ganz besonders gute Ergebnisse erhalten, wenn die zweite Zusammensetzung (B) einen oder mehrere C₁₂-C₃₀-Fettalkohole aus der Gruppe aus Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol), Behenylalkohol (Docosan-1-ol), (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13*Z*)-Docos-13-en-1-ol), Brassidylalkohol ((13*E*)-Docosen-1-ol) 2-Octyl-dodecanol, 2-Hexyl-Dodecanol und/oder 2-Butyl-dodecanol enthält.

Im Rahmen einer ganz besonders bevorzugten Ausführungsform ist ein erfindungsgemäßes Verfahren dadurch gekennzeichnet, dass die zweite Zusammensetzung (B) ein oder mehrere C₁₂-C₃₀-Fettalkohole (B2) aus der Gruppe aus
Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9*Z*)-Octadec-9-en-1-ol (Oleylalkohol),
(9*E*)-Octadec-9-en-1-ol (Elaidylalkohol),
(9*Z*,12*Z*)-Octadeca-9,12-dien-1-ol (Linoleylalkohol),
(9*Z*,12*Z*,15*Z*)-Octadeca-9,12,15-trien-1-ol (Linolenoylalkohol),
Gadoleylalkohol ((9*Z*)-Eicos-9-en-1-ol),
Arachidonalkohol ((5*Z*,8*Z*,11*Z*,14*Z*)-Eicosa-5,8,11,14-tetraen-1-ol),
Erucylalkohol ((13*Z*)-Docos-13-en-1-ol),
Brassidylalkohol ((13*E*)-Docosen-1-ol),
2-Octyl-dodecanol,
2-Hexyl-Dodecanol und/oder
2-Butyl-dodecanol enthält.

Durch Wahl der geeigneten Einsatzmengen an C₁₂-C₃₀-Fettalkoholen (B2) kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere C₁₂-C₃₀-Fettalkohole (B2) in ganz bestimmten Mengenbereichen einzusetzen.

Es ist ganz besonders bevorzugt, wenn die zweite Zusammensetzung (B) - bezogen auf das Gesamtgewicht der Zusammensetzung (B) - ein oder mehrere C₁₂-C₃₀-Fettalkohole (B2) in einer Gesamtmenge von 2,0 bis 50,0 Gew.-%, bevorzugt von 4,0 bis 40,0 Gew.-%, weiter bevorzugt von 6,0 bis 30,0 Gew.-%, noch weiter bevorzugt von 8,0 bis 20,0 Gew.-% und ganz besonders bevorzugt von 10,0 bis 15,0 Gew.-% enthält.

Im Rahmen einer weiteren ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - ein oder mehrere C₁₂-C₃₀-Fettalkohole (B2) in einer Gesamtmenge von 2,0 bis 50,0 Gew.-%, bevorzugt von 4,0 bis 40,0 Gew.-%, weiter bevorzugt von 6,0 bis 30,0 Gew.-%, noch weiter bevorzugt von 8,0 bis 20,0 Gew.-% und ganz besonders bevorzugt von 10,0 bis 15,0 Gew.-% enthält.

Weiterhin als ganz besonders bevorzugten Fettbestandteil (B2) kann die Zusammensetzung (B) auch mindestens ein C₁₂-C₃₀-Fettsäuretriglycerid, der C₁₂-C₃₀-Fettsäuremonoglycerid und/oder C₁₂-C₃₀-Fettsäurediglycerid enthalten. Unter einem C₁₂-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₁₂-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₁₂-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₁₂-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)-Tetracos-15-ensäure].

Unter einem C₁₂-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Ganz besonders gute Ergebnisse wurden erhalten, wenn die Zusammensetzung (B) mindestens ein C₁₂-C₃₀-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Im Rahmen einer ganz besonders bevorzugten Ausführungsform ist ein erfindungsgemäßes Verfahren dadurch gekennzeichnet, dass die zweite Zusammensetzung (B) mindestens ein C₁₂-C₃₀-Fettsäuremonoglycerid (B2) enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Auch durch Wahl der geeigneten Einsatzmengen an C₁₂-C₃₀-Fettsäuremono-, C₁₂-C₃₀-Fettsäuredi- und/oder C₁₂-C₃₀-Fettsäuretriglyceriden kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere C₁₂-C₃₀-Fettsäuremono-, C₁₂-C₃₀-Fettsäuredi- und/oder C₁₂-C₃₀-Fettsäuretriglyceride (B2) in ganz bestimmten Mengenbereichen in der Zusammensetzung (B) einzusetzen.

Im Hinblick auf die Lösung der erfindungsgemäßen Aufgabenstellung hat es sich als ganz besonders bevozugt erwiesen, wenn die zweite Zusammensetzung (B) - bezogen auf das Gesamtgewicht der Zusammensetzung (B) - ein oder mehrere C₁₂-C₃₀-Fettsäuremono-, C₁₂-C₃₀-Fettsäuredi- und/oder C₁₂-C₃₀-Fettsäuretriglyceride (B2) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Im Rahmen einer ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - ein oder mehrere C₁₂-C₃₀-Fettsäuremono-, C₁₂-C₃₀-Fettsäuredi- und/oder C₁₂-C₃₀-Fettsäuretriglyceride (B2) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthält.

Die C₁₂-C₃₀-Fettsäuremono-, C₁₂-C₃₀-Fettsäuredi- und/oder C₁₂-C₃₀-Fettsäuretriglyceride können als alleinige Fettbestandteile (B2) in den Zusammensetzungen (B) eingesetzt werden. Es ist jedoch ganz besonders beovrzugt, mindestens ein C₁₂-C₃₀-Fettsäuremono-, C₁₂-C₃₀-Fettsäuredi- und/oder C₁₂-C₃₀-Fettsäuretriglycerid in Kombination mit mindestens einem C₁₂-C₃₀-Fettalkohol in die Zusammensetzung (B) einzuarbeiten.

Weiterhin als ganz besonders bevorzugten Fettbestandteil (B2) kann die Zusammensetzung (B) auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn die Zusammensetzung (B) mindestens einen Kohlenwasserstoff (B2) enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Verfahren dadurch gekennzeichnet, dass die zweite Zusammensetzung (B) mindestens einen Fettbestandteile (B2) aus der Gruppe der Kohlenwasserstoffe enthält.

Auch durch Wahl der geeigneten Einsatzmengen an Kohlenwasserstoffen kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere Kohlenwasserstoffe in ganz bestimmten Mengenbereichen in der Zusammensetzung (B) einzusetzen.

Im Hinblick auf die Lösung der erfindungsgemäßen Aufgabenstellung hat es sich als ganz besonders bevozugt erwiesen, wenn die zweite Zusammensetzung (B) - bezogen auf das Gesamtgewicht der Zusammensetzung (B) - ein oder mehrere Kohlenwasserstoffe (B2) in einer Gesamtmenge von 0,5 bis 20,0 Gew.-%, bevorzugt 1,0 bis 15,0 Gew.-%, weiter bevorzugt von 1,5 bis 10,0 Gew.-% und ganz besonders bevorzugt von 2,0 bis 8,0 Gew.-% enthielt.

Im Rahmen einer ganz besonders bevorzugten Ausführungsform ist ein erfindungsgemäßes Verfahren dadurch gekennzeichnet, dass die zweite Zusammensetzung (B) - bezogen auf das Gesamtgewicht der Zusammensetzung (B) - einen oder mehrere Kohlenwasserstoffe (B2) in einer Gesamtmenge von 0,5 bis 20,0 Gew.-%, bevorzugt 1,0 bis 15,0 Gew.-%, weiter bevorzugt von 1,5 bis 10,0 Gew.-% und ganz besonders bevorzugt von 2,0 bis 8,0 Gew.-% enthält.

Der oder die Kohlenwasserstoffe können als alleinige Fettbestandteile (B2) in den Zusammensetzungen (B) eingesetzt werden. Es ist jedoch ganz besonders beovrzugt, mindestens einen Kohlenwasserstoff in Kombination mit mindestens einem weiteren Bestandteil in die Zusammensetzungen (B) einzuarbeiten.

Ganz besonders bevorzugt enthält die Zusammensetzung (B) mindestens einen Fettbestandteil (B2) aus der Gruppe der C₁₂-C₃₀-Fettalkohole und mindestens einen weiteren Fettbestandteil aus der Gruppe der Kohlenwasserstoffe.

### Tenside in der Zusammensetzung (B)

Bedingt durch ihren Gehalt an Wasser (B1) und gegebenenfalls Fettbestandteil (B2) kann die Zusammsetzung (B) in Form einer Emulsion vorliegen. Um die Ausbildung der Emulsion weiter zu optimieren, hat es sich als ganz besonders bevorzugt erwiesen, in der Zusammensetzung (B) weiterhin mindestens ein Tensid einzusetzen.

Ganz besonders bevorzugt enthält die Zusammensetzung (B) deshalb zusätzlich mindestens ein Tensid.

Im Rahmen einer weiteren besonders bevorzugten Ausführungsform ist ein erfindungsgemäßes Verfahren dadurch gekennzeichnet, dass die zweite Zusammensetzung (B) mindestens ein Tensid, enthält.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Im Rahmen einer ganz besonders bevorzugten Ausführungsform ist ein erfindungsgemäßes Verfahren dadurch gekennzeichnet, dass die zweite Zusammensetzung (B) mindestens ein nichtionisches Tensid enthält.

Nichtionische Tenside enthalten beipsielsweise als hydrophile Gruppe eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (Tnio-2)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (Tnio-2) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (Tnio-3),

   R⁵CO-NR⁶-[Z] (Tnio-3)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (Tnio-4) wiedergegeben werden:

   R⁷CO-(NR⁸) -CH₂ - [CH(OH)]₄ - CH₂OH (Tnio-4)

   Vorzugsweise werden als Fettsäure-N-alkylpolyhydroxyalkylamide Glucamide der Formel (Tnio-4) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (Tnio-4), die durch reduktive Aminierung von Glucose mit Methylamin und anschließende Acylierung mit Laurinsäure oder C12/14-Kokosfettsäure beziehungsweise einem entsprechenden Derivat erhalten werden. Weiterhin können sich die Polyhydroxyalkylamide auch von Maltose und Palatinose ableiten.

Die Zuckertenside können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.-%.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Besonders gute Ergebnisse wurden erhalten, wenn im erfindungsgemäßen Verfahren eine zweite Zusammensetzung (B) eingesetzt wurde, welche mindestens einen ethoxylierten Fettalkohol mit einem Ethoxylierungsgrad von 80 bis 120 enthielt.

In einer weiteren ganz besonders bevorzugten Ausführungsform ist ein erfindungsgemäßes Verfahren dadurch gekennzeichnet, dass die zweite Zusammensetzung (B) mindestens ein nichtionisches Tensid der Formel (T-I) enthält, 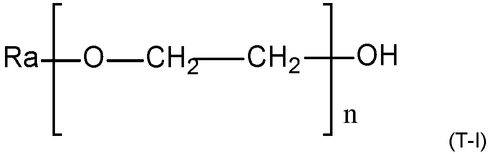 worin Ra für eine gesättigte oder ungesättigte, unverzweigte oder verzweigte C₈-C₂₄-Alkylgruppe, bevorzugt für eine gesättigte, unverzweigte C₁₆- bis C₁₈- Alkylgruppe, steht und n für eine ganze Zahl von 80 bis 120, bevorzugt für eine ganze Zahl von 90 bis 110 und besonders bevorzugt für die Zahl 100, steht.

Ein besonders gut geeignetes nichtionisches Tensid dieses Typs trägt den Handelsnamen Brij S 100 bzw. Brij S 100 PA SG. Hierbei handelt es sich um Stearylalkohol, ethoxyliert mit 100 EO, der von der Firma Croda kommerziell erhältlich ist und die CAS-Nummer 9005-00-9 trägt.

Weiterhin ganz besonders gute Ergebnisse wurden erhalten, wenn im erfindungsgemäßen Verfahren eine zweite Zusammensetzung (B) eingesetzt wurde, welche mindestens einen ethoxylierten Fettalkohole mit einem Ethoxylierungsgrad von 10 bis 40 enthielt.

In einer weiteren ganz besonders bevorzugten Ausführungsform ist ein erfindungsgemäßes Verfahren dadurch gekennzeichnet, dass die zweite Zusammensetzung (B) mindestens ein nichtionisches Tensid der Formel (T-II) enthält, 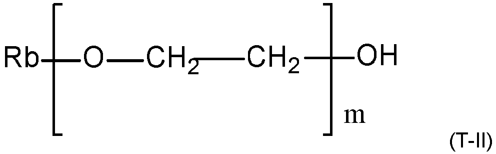 worin
- Rb: für eine gesättigte oder ungesättigte, unverzweigte oder verzweigte C₈-C₂₄-Alkylgruppe, bevorzugt für eine gesättigte, unverzeigte C₁₆- bis C₁₈- Alkylgruppe, steht und
- m: für eine ganze Zahl von 10 bis 40, bevorzugt für eine ganze Zahl von 20 bis 35 und besonders bevorzugt für die Zahl 30, steht.

Bei einem besonders gut geeigneten nichtionischen Tensid dieses Typs handelt es sich um Ceteareth-30. Bei Ceteareth-30 handelt es sich um ein Gemisch aus Cetylalkohol und Stearylalkohol, die jeweils mit 30 Einheiten Ethylenoxid ethoxyliert sind. Das Gemisch aus Cetylalkohol und Stearylalkohol wird als Cetearylalkohol bezeichnet. Ceteareth-30 besitzt die CAS-Nummer 68439-49-6 und ist beispielsweise unter dem Handelsnamen Eumulgin B3 von der Firma BASF käuflich zu erwerben.

Es hat sich als ganz besonders bevorzugt erwiesen, wenn die Zusammensetzung (B) sowohl mindestens ein nichtionisches Tensid der Formel (T-I) als auch mindestens ein nichtionisches Tensid der Formel (T-II) enthält.

### Polymere in der Zusammensetzung (B)

Im Rahmen einer weiteren Ausführungsform kann die im erfindungsgemäßen Verfahren eingesetzte Zusammensetzung (B) auch in Form eines wasserhaltigen Gels konfektioniert werden.

Als weiteren optionalen Bestandteil kann die Zusammensetzung (B) daher auch mindestens ein Polymer, besonders bevorzugt ein verdickendes Polymer enthalten.

Als geeignete Polymere können in diesem Zusammenhang beispielsweise genannt werden:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die Polymere sind in der Zusammensetzung (B) bevorzugt in Mengen von 0,05 bis 10 Gew.-%, bezogen auf das gesamte Mittel, enthalten. Mengen von 0,1 bis 5 Gew.-% sind besonders bevorzugt.

Im Rahmen einer weiteren ganz besonders bevorzugten Ausführungsform ist ein erfindungsgemäßes Verfahren dadurch gekennzeichnet, dass die zweite Zusammensetzung (B) mindestens ein verdickendes Polymer, bevorzugt mindestens einen Celluloseether ausgewählt aus der Gruppe aus Hydroxyethylcellulose, Hydroxypropylcellulose und Methylhydroxypropylcellulose, enthält.

### Lösungsmittel in der Zusammensetzung (B)

Weitere zu dieser Erfindung führende Arbeiten haben gezeigt, dass auch der Einsatz mindestens eines protischen Lösungsmittels in der Zusammensetzung (B) bei Kontakt mit der Zusammensetzung (A) die Reaktionsgeschwindigkeit der C₁-C₆-Alkoxy-Silane reduzieren kann. Aus diesem Grund kann der Zusammensetzung (B) zusätzlich auch noch mindestens ein Lösungsmittel hinzugesetzt werden.

Protische Lösungsmittel besitzen mindestens eine Hydroxy-Gruppe. Ohne auf diese Theorie festgelegt zu sein, wird vermutet, dass die Lösungmittel über ihre Hydroxy-Gruppe(n) auch mit den C₁-C₆-Alkoxysilanen reagieren können, die Reaktion zwischen Lösungsmittel und C₁-C₆-Alkoxysilanen jedoch langsamer abläuft als die analoge Reaktion zwischen Wasser und C₁-C₆-Alkoxysilanen. In Summe wird auf diesem Wege die Hydrolyse- und/oder die Kondensationsreaktion der C₁-C₆-Alkoxy-silane vermindert.

Als gut geeignete Lösungsmittel können beispielsweise 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und/oder Benzylalkohol genannt werden.

In einer weiteren ganz besonders bevorzugten Ausführungsform ist ein erfindungsgemäßes Verfahren dadurch gekennzeichnet, dass die zweite Zusammensetzung (B) mindestens ein Lösungsmittel aus der Gruppe aus 1,2-Propylenglycol, 1,3-Propylenglycol, Ethylenglycol, 1,2-Butylenglycol, Dipropylenglycol, Ethanol, Isopropanol, Diethylenglycolmonoethylether, Glycerin, Phenoxyethanol und/oder Benzylalkohol enthält.

Zusammensetzungen (B), die 1,2-Propylenglycol als Lösungsmittel enthalten, sind ganz besonders bevorzugt.

1,2-Propylenglycol wird alternativ auch als 1,2-Propandiol bezeichnet wird und trägt die CAS-Nummern 57-55-6 [(RS)-1,2-Dihydroxypropan], 4254-14-2 [(R)-1,2-Dihydroxypropan] und 4254-15-3 [(S)-1,2-Dihydroxypropan]. Ethylenglycol wird alternativ auch als 1,2-Ethandiol bezeichnet und trägt die CAS-Nummer 107-21-1.Glycerin wird alternativ auch als 1,2,3-Propantriol bezeichnet und trägt die CAS-Nummer 56-81-5. Phenoxyethanol besitzt die Cas-Nummer 122-99-6.

Alle zuvor beschriebenen Lösungsmittel sind bei verschiedenen Chemikalien-Lieferanten wie beispielsweise Aldrich oder Fluka kommerziell erhältlich.

Durch Verwendung der vorgenannten Lösungsmittel in geeigneten Einsatzmengen, kann die Geschwindigkeit der von den C₁-C₆-Alkoxy-Silanen ausgehenden Filmbildung besonders stark mitbestimmt werden. Aus diesem Grund hat es sich als ganz besonders bevorzugt erwiesen, ein oder mehrere Lösungsmittel in ganz bestimmten Mengenbereichen einzusetzen.

Es ist ganz besonders bevorzugt, wenn die zweite Zusammensetzung (B) - bezogen auf das Gesamtgewicht der Zusammensetzung (B) - ein oder mehrere Lösungsmittel in einer Gesamtmenge von 1,0 bis 35,0 Gew.-%, bevorzugt von 4,0 bis 25,0 Gew.-%, weiter bevorzugt von 8,0 bis 20,0 Gew.-%, und ganz besonders bevorzugt von 10,0 bis 15,0 Gew.-% enthält.

Es ist ganz besonders bevorzugt, wenn die zweite Zusammensetzung (B) - bezogen auf das Gesamtgewicht der Zusammensetzung (B) - ein oder mehrere Lösungsmittel aus der Gruppe aus 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und/oder Benzylalkohol in einer Gesamtmenge von 1,0 bis 35,0 Gew.-%, bevorzugt von 4,0 bis 25,0 Gew.-%, weiter bevorzugt von 8,0 bis 20,0 Gew.-%, und ganz besonders bevorzugt von 10,0 bis 15,0 Gew.-% enthält.

### Weitere kosmetische Inhaltsstoffe in der Zusammensetzung (B)

Zusätzlich zu den bereits zuvor beschriebenen ganz besonderen bevorzugten Inhaltsstoffen kann die Zusammensetzung (B) darüber hinaus auch noch einen oder mehrere weitere kosmetische Inhaltsstoffe enthalten.

Bei den fakultativ in der Zusammensetzung (B) einsatzbaren kosmetischen Inhaltsstoffen kann es sich um alle geeigneten Bestandteile handeln, um dem Mittel weitere positive Eigenschaften zu verleihen. Beispielsweise können in der Zusammensetzung (A) ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der C₈-C₃₀-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate enthalten sein.

Handelt es sich bei dem erfindungsgemäßen Verfahren um ein Verfahren zur Färbung von keratinischem Material, so kann die Zusammensetzung (B) ganz besonders bevorzugt mindestens eine färbende Verbindung aus der Gruppe der Pigmente und/oder der direktziehenden Farbstoffe enthalten.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

### pH-Werte der Zusammensetzungen im Verfahren

In weiteren Versuchen hat sich herausgestellt, dass die pH-Werte der Zusammensetzungen (A) und/oder (B) einen Einfluss auf die bei der Anwendung ablaufenden zuvor beschriebenen Hydrolyse- bzw. Kondensationsreaktionen nehmen können. Hierbei wurde gefunden, dass insbesondere alkalische pH-Werte die Kondensation auf der Stufe der Oligomere anhalten. Je saurer das Reaktionsgemisch ist, desto stärker scheint die Kondensation abzulaufen und desto höher ist das Molekulargewicht der bei der Kondensation entstehenden Silan-Kondensate. Aus diesem Grund ist es bevorzugt, dass die Zusammensetzungen (A) und/oder (B) einen pH-Wert von 5,0 bis 12,0, bevorzugt von 6,0 bis 11,5, weiter bevorzugt von 8,5 bis 11,0 und ganz besonders bevorzugt von 9,0 bis 11,0 besitzen.

Der Wassergehalt der Zusammensetzung (A) liegt bei maximal 10,0 Gew.-% und wird bevorzugt noch geringer eingestellt. Im Rahmen einiger Ausführungsformen kann auch der Wassergehalt der Zusammensetzung (B) gering gewählt sein. Insbesondere bei Zusammensetzungen mit sehr geringem Wassergehalt kann sich die Messung des pH-Wertes mit den üblichen aus dem Stand der Technik bekannten Methoden (pH-Wert Messung mittels Glaselektroden über Einstabmessketten oder aber über pH-Indikator-Papier) als schwierig erweisen. Aus diesem Grund handelt es sich bei den erfindungsgemäßen pH-Werten um die Werte, die nach Vermischen oder Verdünnung der Zubereitung im Gewichtsverhältnis 1:1 mit destilliertem Wasser erhalten wurden.

Der entsprechende pH-Wert wird dementsprechend gemessen, nachdem beispielsweise 50 g der erfindungsgemäßen Zusammensetzung mit 50 g destilliertem Wasser vermischt wurden.

In einer weiteren ganz besonders bevorzugten Ausführungsform ist ein erfindungsgemäßes Verfahren, dadurch gekennzeichnet, dass die Zusammensetzung (A) und/oder (B) nach der Verdünnung mit destilliertem Wasser im Gewichtsverhältnis 1:1 einen pH-Wert von 5,0 bis 12,0, bevorzugt von 6,0 bis 11,5, weiter bevorzugt von 8,5 bis 11,0 und ganz besonders bevorzugt von 9,0 bis 11,0 besitzt.

Zur Einstellung dieses alkalischen pH-Wertes kann es erforderlich werden, der Reaktionsmischung ein Alkalisierungsmittel und/oder Acidifizierungsmittel zuzusetzen. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Aminosäuren eingesetzt werden.

Alkanolamine können ausgewählt werden aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch anorganische Alkalisierungsmittel eingesetzt werden. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierungsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

### Anwendung der Zusammensetzungen (A) und (B)

Das erfindungsgemäße Verfahren umfasst die Anwendung der Zusammensetzungen (A), (B) und (C) auf dem keratinischen Material. Wesentlich für das Verfahren ist, dass die Zusammensetzungen (A) und (B) auf dem keratinischen Material miteinander in Kontakt kommen. Wie bereits zuvor beschrieben kann dieser Kontakt entweder durch vorheriges Abmischen von (A) und (B) oder durch sukzessives Auftragen von (A) und (B) auf dem Keratinmaterial entstehen. Der Kontakt der Bestandteile von (A) und (B) kann damit bereits vor der Anwendung in der Formulierung hergestellt werden, oder aber während der Anwendung auf dem Keratinmatieral selbst erfolgen.

Die zu dieser Erfindung führenden Arbeiten haben gezeigt, dass die Wasser (B1) und Aldehyde (B2) enthaltende Zusammensetzung (B) insbesondere dann optimalen Einfluss auf den wasserarmen Silan-Blend (d.h. auf die Zusammensetzung (A)) nehmen kann, wenn die Zusammensetzungen (A) und (B) vor der Anwendung miteinander vermischt wurden.

Dieses Vermischen kann beispielsweise durch Verrrühren oder Verschütteln erfolgen. Ganz gesonders vorteilhaft ist es, die beiden Zusammensetzungen (A) und (B) getrennt in zwei Containern zu konfektionieren, und vor der Anwendung dann die gesamte Menge der Zusammensetzung (A) aus ihrem Container in den Container zu überführen, in dem sich die zweite Zusammensetzung (B) befindet.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde.

Die beiden Zusammensetzungen (A) und (B) können in unterschiedlichen Mengenverhältnissen miteinander vermischt werden.

Besonders bevorzugt wird die Zusammensetzung (A) in Form eines relativ hoch konzentrierten, wasserarmen Silan-Blends eingesetzt, der durch Vermischen mit der Zusammensetzung (B) quasi verdünnt wird. Aus diesem Grund ist es ganz besonders bevorzugt, die Zusammensetzung (A) mit einem Gewichtsüberschuss an Zusammensetzung (B) zu vermischen. So kann beispielsweise 1 Gewichtsanteil (A) mit 20 Gewichtsanteilen (B) vermischt werden, oder 1 Gewichtsanteil (A) wird mit 10 Gewichtsanteilen (B) vermischt, oder aber 1 Gewichtsanteil (A) wird mit 5 Gewichtsanteilen (B) vermischt.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) und der zweiten Zusammensetzung (B) in einem Mengenverhältnis (A)/(B) von 1:5 bis 1:20 hergestellt wurde.

Prinzipiell ist es aber auch möglich, die Zusammensetzung (A) im Verhältnis zur Zusammensetzung (B) in einem Gewichtsüberschuss einzusetzen. So können auch beipsielsweise 20 Gewichtsanteile (A) mit 1 Gewichtsanteil (B) vermischt werden, oder 10 Gewichtsanteile (A) werden mit 1 Gewichtsanteil (B) vermischt, oder aber 5 Gewichtsanteile (A) werden mit 1 Gewichtsanteil (B) vermischt.

Weiterhin ist es auch denkbar, die Zusammsetzungen (A) und (B) sukzessive auf dem keratinischen Material anzuwenden, so dass der Kontakt von (A) und (B) erst auf dem keratinischen Material zustande kommt. Im Rahmen dieser Ausführungsform erfolgt zwischen der Anwendung der Zusammensetzungen (A) und (B) bevorzugt keine Wäsche des Keratinmatierals, d.h. keine Behandlung des Keratinmatierals mit Wasser bzw. Wasser und Tensiden.

Bei der dritten Zusammensetzung (C) handelt es sich um eine Zusammensetzung, die mindestens eine farbgebende Verbindung aus der Gruppe der Pigmente und/oder der direktziehenden Farbstoffe enthält.

Bei Anwendung der drei Zusammensetzungen (A), (B) und (C) sind verschiedene Ausführungsformen erfindungsgemäß.

Im Rahmen einer Ausführungsform ist es ganz besonders bevorzugt, vor der Anwendung eine Abmischung aus den drei Zusammensetzungen (A), (B) und (C) herzustellen und dann dieses Gemisch dann auf dem Keratinmaterial anzuwenden.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) mit der zweiten Zusammensetzung (B) und einer dritten Zusammensetzung (C) erhalten wurde,
wobei die dritte Zusammensetzung (C) mindestens eine farbgebende Verbindung aus der Gruppe der Pigmente und/oder der direktziehenden Farbstoffe enthält.

Bei Färbung des Keratinmaterials kann es ebenfalls ganz besonders bevorzugt sein, unmittelbar vor der Anwendung durch Vermischen der ersten Zusammensetzung (A) und der zweiten Zusammensetzung (B) eine Mischung herzustellen und diese Mischung aus (A) und (B) auf dem Keratinmaterial anzuwenden. Die dritte Zusammensetzung (C) mit den farbgebenden Verbindungen kann dann im Anschluss daran auf das Keratinmaterial gegeben werden.

Im Rahmen einer ganz besonders bevorzugten Ausführungsform ist ein erfindungsgemäßes Verfahren dadurch gekennzeichnet, dass auf dem keratinischen Material eine Zusammensetzung angewendet wird, die unmittelbar vor der Anwendung durch Vermischen der ersten Zusammensetzung (A) mit der zweiten Zusammensetzung (B) erhalten wurde, und im Anschluss daran die die Zusammensetzung (C) auf dem keratinischen Material angewendet wird.

Mit anderen Worten ist ein ganz besonders bevorzugtes erfindungsgemäßes Verfahren dadurch gekennzeichnet, dass in einem ersten Schritt auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde, und in einem zweiten Schritt auf dem keratinischen Material die weitere Zusammensetzung (C) angewendet wird.

Zusätzlich zu den Zusammensetzungen (A), (B) und (C) kann im erfindungsgemäßen Verfahren auch noch eine weitere bzw. eine vierte Zusammensetzung (D) auf dem Keratinmaterial appliziert werden. Die Anwendung der Zusammensetzung (D) erfolgt besonders bevorzugt in einem Färbevefahren, um die zuvor erhaltenen Färbungen nochmals zu versiegeln. Für diese Versiegelung kann die Zusammensetzug (D) beispeilsweise mindestens ein filmbildendes Polymer enthalten.

Mit anderen Worten weiterhin ganz besonders bevorzugt ist ein erfindungsgemäßes Verfahren bei dem auf dem keratinischen Material angewendet wird
- eine weitere Zusammensetzung (D), die enthält mindestens eine filmbildendes Polymer.

### Farbgebende Verbindungen

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte farbgebende Verbindungen aus der Gruppe der Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Im Rahmen einer ganz besonders bevorzugten Ausführungsform ist ein erfindungsgemäßes Verfahren dadurch gekennzeichnet, dass die Zusammensetzung (C) mindestens eine farbgebende Verbindung aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist die erfindungsgemäße Zusammensetzung (C) dadurch gekennzeichnet, dass sie mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbgebenden Verbindungen auf Mica- oder Glimmerbasis, die mit mindestens einem Metalloxid und/oder einem Metalloxychlorid beschichtet sind.

In einer weiteren bevorzugten Ausführungsform ist eine erfindungsgemäße Zusammensetzung (C) dadurch gekennzeichnet, dass sie mindestens eine farbgebende Verbindung enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel bzw. die erfindungsgemäße Zubereitung auch ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass die Zusammensetzung (C) mindestens eine farbgebende Verbindung aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Zur Färbung des Keratinmaterials können auch Pigmente mit einer bestimmten Formgebung eingesetzt worden sein. Beispielsweise kann ein Pigment auf Basis eines lamellaren und/oder eines lentikularen Substratplättchens eingesetzt werden. Weiterhin ist auch die Färbung auf Basis eines Substratplättchens möglich, welches ein Vakuum metallisiertes Pigment umfasst.

Die Substratplättchen dieses Typs weisen eine durchschnittliche Dicke von höchstens 50 nm, vorzugsweise weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Substratplättchen sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf.

Durch die geringe Dicke der Substratplättchen weist das Pigment ein besonders hohes Deckvermögen auf.

Die Substratplättchen sind monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können. Die Substratplättchen sind vorzugsweise homogen aufgebaut, d.h. dass innerhalb der Plättchen kein Konzentrationsgradient auftritt. Insbesondere sind die Substratplättchen nicht schichtartig aufgebaut und weisen keine darin verteilten Teilchen oder Partikel auf.

Die Größe des Substratplättchens kann auf den jeweiligen Anwendungszweck, insbesondere dem gewünschten Effekt auf dem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750, beträgt. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen können aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus Metall(legierung)en aufgebaut.

Als Metall kommt jedes für metallische Glanzpigmente geeignete Metall in Betracht. Derartige Metalle sind unter anderem Eisen und Stahl, sowie alle luft- und wasserbeständigen (Halb)metalle wie beispielsweise Platin, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Lamellare Substratplättchen zeichnen sich durch einen unregelmäßig strukturierten Rand aus und werden aufgrund ihres Erscheinungsbildes auch als "cornflakes" bezeichnet.

Aufgrund ihrer unregelmäßigen Struktur erzeugen Pigmente auf der Basis von lamellaren Substratplättchen einen hohen Anteil an Streulicht. Außerdem decken die Pigmente auf der Basis von lamellaren Substratplättchen die vorhandene Farbe eines keratinischen Materials nicht vollständig ab und es können beispielsweise Effekte analog zu einer natürlichen Ergrauung erzielt werden.

Lentikulare (= linsenförmige) Substratplättchen weisen einen im Wesentlichen regelmäßigen runden Rand auf und werden aufgrund ihres Erscheinungsbildes auch als "silverdollars" bezeichnet. Aufgrund ihrer regelmäßigen Struktur überwiegt bei Pigmenten auf Basis von lentikularen Substratplättchen der Anteil des reflektierten Lichts.

Vakuum metallisierte Pigmente *(vacuum metallized pigments,* VMP) können beispielsweise durch das Freisetzen von Metallen, Metalllegierungen oder Metalloxiden von entsprechend beschichteten Folien gewonnen werden. Sie zeichnen sich durch eine besonders geringe Dicke der Substratplättchen im Bereich von 5 bis 50 nm und durch eine besonders glatte Oberfläche mit erhöhter Reflektivität aus. Substratplättchen, welche ein im Vakuum metallisiertes Pigment umfassen, werden im Rahmen dieser Anmeldung auch als VMP-Substratplättchen bezeichnet. VMP-Substratplättchen aus Aluminium können beispielsweise durch Freisetzen von Aluminium von metallisierten Folien gewonnen werden.

Die Substratplättchen aus Metall oder Metalllegierung können passiviert sein, beispielsweise durch Eloxieren (Oxidschicht) oder Chromatieren.

Unbeschichtete lamellare, lentikulare und/oder VPM-Substratplättchen, insbesondere solche aus Metall oder Metalllegierung, reflektieren das einfallende Licht in hohem Maße und erzeugen einen Hell-Dunkel-Flop, aber keinen Farbeindruck.

Ein Farbeindruck kann beispielsweise aufgrund optischer Interferenzeffekte erzeugt werden. Derartige Pigmente können auf mindestens einfach beschichteten Substratplättchen beruhen. Diese zeigen Interferenzeffekte durch Überlagerung von verschieden gebrochenen und reflektierten Lichtstrahlen.

Entsprechend sind bevorzugte Pigmente, Pigmente auf Basis eines beschichteten lamellaren Substratplättchens. Das Substratplättchen weist vorzugsweise mindestens eine Beschichtung B aus einem hochbrechenden Metalloxid mit einer Beschichtungsdicke von mindestens 50 nm auf. Zwischen der Beschichtung B und der Oberfläche des Substratplättchens befindet sich vorzugsweise noch eine Beschichtung A. Gegebenenfalls befindet sich auf der Schicht B eine weitere Beschichtung C, die von der darunterliegenden Schicht B verschieden ist.

Als Materialien für die Beschichtungen A, B und C eignen sich alle Substanzen, die filmartig und dauerhaft auf die Substratplättchen aufgebracht werden können und, im Fall der Schichten A und B, die erforderlichen optischen Eigenschaften aufweisen. Allgemein ist eine Beschichtung eines Teils der Oberfläche der Substratplättchen ausreichend, um ein Pigment mit einem glänzenden Effekt zu erhalten. So können beispielsweise lediglich die obere und/oder untere Seite der Substratplättchen beschichtet sein, wobei die Seitenfläche(n) ausgespart sind. Vorzugsweise ist die gesamte Oberfläche der gegebenenfalls passivierten Substratplättchen, einschließlich der Seitenflächen, von Beschichtung B bedeckt. Die Substratplättchen sind also vollständig von Beschichtung B umhüllt. Dies verbessert die optischen Eigenschaften des Pigments und erhöht die mechanische und chemische Belastbarkeit der Pigmente. Das Vorstehende gilt auch für die Schicht A und vorzugsweise auch für die Schicht C, falls vorhanden.

Obwohl jeweils mehrere Beschichtungen A, B und/oder C vorhanden sein können, weisen die beschichteten Substratplättchen vorzugsweise jeweils nur eine Beschichtung A, B und, falls vorhanden, C auf.

Die Beschichtung B ist aus mindestens einem hochbrechenden Metalloxid aufgebaut. Hochbrechende Materialien weisen einen Brechungsindex von mindestens 1,9, bevorzugt mindestens 2,0 und besonders bevorzugt mindestens 2,4 auf. Vorzugsweise umfasst die Beschichtung B mindestens 95 Gew.-%, besonders bevorzugt mindestens 99 Gew.-% an hochbrechenden Metalloxid(en).

Die Beschichtung B weist eine Dicke von mindestens 50 nm auf. Vorzugsweise beträgt die Dicke von Beschichtung B nicht mehr als 400 nm, besonders bevorzugt höchstens 300 nm.

Für Beschichtung B geeignete hochbrechende Metalloxide sind vorzugsweise selektiv lichtabsorbierende (d.h. farbige) Metalloxide, wie beispielsweise Eisen(III)oxid (α- und γ-Fe2O3, rot), Cobalt(II)oxid (blau), Chrom(III)oxid (grün),Titan(III)oxid (blau, liegt üblicherweise im Gemisch mit Titanoxynitriden und Titannitriden vor) und Vanadium(V)oxid (orange) sowie deren Gemische. Es eignen sich auch farblose hochbrechende Oxide wie Titandioxid und/oder Zirkonoxid.

Beschichtung B kann einen selektiv absorbierenden Farbstoff enthalten, vorzugsweise 0,001 bis 5 Gew.-%, besonders bevorzugt 0,01 bis 1 Gew.-%, jeweils bezogen auf die Gesamtmenge der Beschichtung B. Geeignet sind organische und anorganische Farbstoffe, die sich stabil in eine Metalloxidbeschichtung einbauen lassen.

Die Beschichtung A weist vorzugsweise mindestens ein niedrigbrechendes Metalloxid und/oder Metalloxidhydrat auf. Vorzugsweise umfasst Beschichtung A mindestens 95 Gew.-%, besonders bevorzugt mindestens 99 Gew.-% niedrigbrechendes Metalloxid(hydrat). Niedrigbrechende Materialien weisen einen Brechungsindex von höchstens 1,8, bevorzugt höchstens 1,6 auf.

Zu den niedrigbrechenden Metalloxiden, die für die Beschichtung A geeignet sind, zählen beispielsweise Silicium(di)oxid, Siliciumoxidhydrat, Aluminiumoxid, Aluminiumoxidhydrat, Boroxid, Germaniumoxid, Manganoxid, Magnesiumoxid und deren Gemische, wobei Siliciumdioxid bevorzugt ist. Die Beschichtung A weist bevorzugt eine Dicke von 1 bis 100 nm, besonders bevorzugt 5 bis 50 nm, insbesondere bevorzugt 5 bis 20 nm, auf.

Vorzugsweise beträgt der Abstand zwischen der Oberfläche der Substratplättchen und der inneren Oberfläche von Beschichtung B höchstens 100 nm, besonders bevorzugt höchstens 50 nm, insbesondere bevorzugt höchstens 20 nm. Dadurch, dass die Dicke von Beschichtung A und somit der Abstand zwischen der Oberfläche der Substratplättchen und Beschichtung B im oben angegebenen Bereich liegt, kann sichergestellt werden, dass die Pigmente ein hohes Deckvermögen aufweisen.

Weist das Pigment auf Basis eines lamellaren Substratplättchens nur eine Schicht A auf, ist es bevorzugt, dass das Pigment ein lamellares Substratplättchen aus Aluminium und eine Schicht A aus Siliciumdioxid aufweist. Weist das Pigment auf Basis eines lamellaren Substratplättchens eine Schicht A und eine Schicht B auf, ist es bevorzugt, dass das Pigment ein lamellares Substratplättchen aus Aluminium, eine Schicht A aus Siliciumdioxid und eine Schicht B aus Eisenoxid aufweist.

Gemäß einer bevorzugten Ausführungsform weisen die Pigmente eine weitere Beschichtung C aus einem Metalloxid(hydrat), die von der darunterliegenden Beschichtung B verschieden ist, auf. Geeignete Metalloxide sind beispielsweise Silicium(di)oxid, Siliciumoxidhydrat, Aluminiumoxid, Aluminiumoxidhydrat, Zinkoxid, Zinnoxid, Titandioxid, Zirkonoxid, Eisen(III)oxid und Chrom(III)oxid. Bevorzugt ist Siliciumdioxid.

Die Beschichtung C weist vorzugsweise eine Dicke von 10 bis 500 nm, besonders bevorzugt 50 bis 300 nm auf. Durch das Vorsehen der Beschichtung C, beispielsweise auf Basis von TiO₂, kann eine bessere Interferenz erzielt werden, wobei ein hohes Deckvermögen gewährleistet bleibt.

Die Schichten A und C dienen insbesondere als Korrosionsschutz als auch der chemischen und physikalischen Stabilisierung. Besonders bevorzugt enthalten die Schichten A und C sind Siliciumdioxid oder Aluminiumoxid, die nach dem Sol-Gel-Verfahren aufgebracht werden. Dieses Verfahren umfasst das Dispergieren der unbeschichteten lamellaren Substratplättchen oder der bereits mit Schicht A und/oder Schicht B beschichteten lamellaren Substratplättchen in einer Lösung eines Metallalkoxids wie Tetraethylorthosilikat oder Aluminiumtriisopropanolat (üblicherweise in einer Lösung von organischem Lösungsmittel oder einer Mischung von organischem Lösungsmittel und Wasser mit mindestens 50 Gew.-% organisches Lösungsmittel wie ein C1 bis C4-Alkohol), und Zugabe einer schwachen Base oder Säure zur Hydrolysierung des Metallalkoxids, wodurch ein Film des Metalloxids auf der Oberfläche der (beschichteten) Substratplättchen entsteht.

Die Schicht B kann beispielsweise durch hydrolytische Zersetzung einer oder mehrerer organischer Metallverbindungen und/oder durch Fällung eines oder mehrerer gelöster Metallsalze sowie eine ggf. anschließende Nachbehandlung (zum Beispiel Überführen einer gebildeten hydroxidhaltigen Schichten in die Oxidschichten durch Tempern) hergestellt werden.

Obwohl jede der Beschichtungen A, B und/oder C aus einem Gemisch von zwei oder mehreren Metalloxid(hydrat)en aufgebaut sein kann, ist jede der Beschichtungen vorzugsweise aus einem Metalloxid(hydrat) aufgebaut.

Die Pigmente auf Basis von beschichteten lamellaren bzw. lentikularen Substratplättchen bzw. die Pigmente auf Basis von beschichteten VMP-Substratplättchen weisen vorzugsweise eine Dicke von 70 bis 500 nm, besonders bevorzugt 100 bis 400 nm, insbesondere bevorzugt 150 bis 320 nm, beispielsweise 180 bis 290 nm, auf. Aufgrund der geringen Dicke der Substratplättchen weist das Pigment ein besonders hohes Deckvermögen auf. Die geringe Dicke der beschichteten Substratplättchen wird insbesondere dadurch erzielt, dass die Dicke der unbeschichteten Substratplättchen gering ist, aber auch dadurch, dass die Dicken der Beschichtungen A und, falls vorhanden, C auf einen möglichst kleinen Wert eingestellt werden. Die Dicke von Beschichtung B bestimmt den Farbeindruck des Pigments.

Die Haftung und Abriebbeständigkeit von Pigmenten auf Basis von beschichteten Substratplättchen im keratinischen Material kann deutlich erhöht werden, in dem die äußerste Schicht, je nach Aufbau Schicht A, B oder C, zusätzlich durch organische Verbindung wie Silane, Phosphorsäureester, Titanate, Borate oder Carbonsäuren modifiziert wird. Dabei sind die organischen Verbindungen an die Oberfläche der äußersten, vorzugsweise Metalloxid-haltigen, Schicht A, B oder C gebunden. Die äußerste Schicht bezeichnet die Schicht, die räumlich am weitesten von dem lamellaren Substratplättchen entfernt ist. Bei den organischen Verbindungen handelt es sich vorzugsweise um funktionelle Silanverbindungen, die an die Metalloxid-haltige Schicht A, B oder C binden können. Hierbei kann es sich entweder um mono- als auch um bifunktionelle Verbindungen handeln. Beispiele für bifunktionelle organische Verbindungen sind Methacryloxypropenyltrimethoxysilan, 3-Methacryloxypropyltrimethoxysilan, 3- Acryloxypropyltrimethoxysilan, 2-Acryloxyethyltrimethoxysilan, 3-Methacryloxy- propyltriethoxysilan, 3-Acryloxypropyltrimethoxysilan, 2-Methacryloxyethyl- triethoxysilan, 2-Acryloxyethyltriethoxysilan, 3-Methacryloxypropyltris(methox-yethoxy)silan, 3-Methacryloxypropyltris(butoxyethoxy)silan, 3-Methacryloxy-propyltris(propoxy)silan, 3-Methacryloxypropyltris(butoxy)silan, 3-Acryloxy-propyltris(methoxyethoxy)silan, 3-Acryloxypropyltris(butoxyethoxy)silan, 3-Acryl-oxypropyltris(butoxy)silan, Vinyltrimethoxysilan, Vinyltriethoxysilan, Vinylethyl- dichlorsilan, Vinylmethyldiacetoxysilan, Vinylmethyldichlorsilan, Vinylmethyldiethoxysilan, Vinyltriacetoxysilan, Vinyltrichlorsilan, Phenylvinyldiethoxysilan, oder Phenylallyldichlorsilan. Des Weiteren kann eine Modifizierung mit einem monofunktionellen Silan, insbesondere eines Alkylsilans oder Arylsilans, erfolgen. Dieses weist nur eine funktionelle Gruppe auf, welche kovalent an die Oberfläche Pigments auf Basis von beschichteten lamellaren Substratplättchens (d.h. an die äußerste Metalloxid-haltige Schicht) oder, bei nicht ganz vollständiger Bedeckung, an die Metalloberfläche anbinden kann. Der Kohlenwasserstoffrest des Silans weist vom Pigment weg. Je nach der Art und Beschaffenheit des Kohlenwasserstoffrests des Silans wird ein unterschiedlicher Grad der Hydrophobierung des Pigments erreicht. Beispiele für solche Silane sind Hexadecyltrimethoxysilan, Propyltrimethoxysilan, etc. Besonders bevorzugt sind Pigmente auf Basis von Siliciumdioxid-beschichteten Aluminiumsubstratplättchen mit einem monofunktionellen Silan oberflächenmodifiziert. Besonders bevorzugt sind Octyltrimethoxysilan, Octyltriethoxysilan, Hecadecyltrimethoxysilan sowie Hecadecyltriethoxysilan. Durch die veränderten Oberflächeneigenschaften / Hydrophobierung kann eine Verbesserung bezüglich Haftung, Abriebfestigkeit und Ausrichtung in der Anwendung erzielt werden.

Geeignete Pigmente auf Basis eines lamellaren Substratplättchens umfassen beispielsweise die Pigmente der Reihe VISIONAIRE von Eckart.

Pigmente auf Basis eines lentikularen Substratplättchens sind beispielsweise unter der Bezeichnung Alegrace^{®} Gorgeous von der Firma Schlenk Metallic Pigments GmbH erhältlich.

Pigmente auf Basis eines Substratplättchens, welches ein Vakuum metallisiertes Pigment umfasst, sind beispielsweise unter der Bezeichnung Alegrace^{®} Marvelous oder Alegrace^{®} Aurous von der Firma Schlenk Metallic Pigments GmbH erhältlich.

Das oder die Pigmente können in einer Menge von 0,001 bis 20 Gew.-%, insbesondere von 0,05 bis 5 Gew.-%, jeweils bezogen auf das Gesamtgewicht des erfindungsgemäßen Mittels bzw. der Zubereitung, eingesetzt werden.

Als farbgebende Verbindungen können die erfindungsgemäßen Mittel auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L. Besonders bevorzugt besitzen die direktziehenden Farbstoffe im Sinne der vorliegenden Erfindung eine Löslichkeit in Wasser (760 mmHg) bei 25 °C von mehr als 1,5 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung mindestens einen anionischen, kationischen und/oder nichtionischen direktziehenden Farbstoff enthält.

In einer weiteren bevorzugten Ausführungsform ist ein erfindungsgemäßes Verfahren dadurch gekennzeichnet, dass die Zusammensetzung (C) mindestens eine farbgebende Verbindung aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735),

Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Weiterhin können auch thermochrome Farbstoffe eingesetzt werden. Thermochromie beinhaltet die Eigenschaft eines Materials, in Abhängigkeit der Temperatur reversibel oder irreversibel seine Farbe zu ändern. Dies kann sowohl durch Änderung der Intensität und/oder des Wellenlängenmaximums erfolgen.

Schließlich ist es auch möglich, photochrome Farbstoffe einzusetzen. Photochromie beinhaltet die Eigenschaft eines Materials, in Abhängigkeit der Bestrahlung mit Licht, insbesondere UV-Licht, reversibel oder irreversibel seine Farbe zu ändern. Dies kann sowohl durch Änderung der Intensität und/oder des Wellenlängenmaximums erfolgen.

### Filmbildende Polymere

Die zuvor beschriebenen Zubereitungen, insbesondere die Zubereitungen (B), (C) und (D), ganz besonders bevorzugt die Zubereitung (D), können mindestens ein filmbildendes Polymer enthalten.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Die filmbildenden Polymere können hydrophil oder hydrophob sein.

Im Rahmen einer ersten Ausführungsform kann es bevorzugt sein, in der Zubereitung (B), (C) und/oder (D), ganz besonders in der Zubereitung (D), mindestens ein hydrophobes, filmbildendes Polymer einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein erfindungsgemäßes Mittel dadurch gekennzeichnet, dass es mindestens ein filmbildendes, hydrophobes Polymer enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende hydrophobe Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Me-thacrylate Copolymer), Aculyne^{®} 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Es konnten auch dann intensive und waschechte Färbungen erhalten werden konnten, wenn die Zubereitung (B), (C) und/oder (D), ganz besonders in die Zubereitung (D), mindestens ein filmbildendes Polymer enthielt, das ausgewählt wurde aus der Gruppe der der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

In einer weiteren bevorzugten Ausführungsform ist ein erfindungsgemäßes Verfahren dadurch gekennzeichnet, dass die Zubereitung (B), (C) und/oder (D), ganz besonders die Zubereitung (D), mindestens ein filmbildendes Polymer enthält, das ausgewählt ist aus der Gruppe der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

Im Rahmen einer ersten Ausführungsform kann es bevorzugt sein, in der Zubereitung (B), (C) und/oder (D), ganz besonders in der Zubereitung (D), mindestens ein hydrophiles, filmbildendes Polymer einzusetzen.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint markoskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummen, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein erfindungsgemäßes Mittel dadurch gekennzeichnet, dass es mindestens ein filmbildendes, hydrophiles Polymer enthält, das ausgewählt ist aus der Gruppe aus Polyvinylpyrrolidon (PVP) und den Copolymeren des Polyvinylpyrrolidons.

Es ist weiterhin bevorzugt, wenn das erfindungsgemäße Mittel als filmbildendes, hydrophiles Polymer Polyvinylpyrrolidon (PVP) enthält. Überraschenderweise war auch die Waschechtheit der Färbungen, die mit mit PVP-haltigen Mitteln (b9 erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskole K 90 oder Luviskole K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren aus der Gruppe der Copolymere des Polyvinylpyrrolidons hat ebenfalls zu besonders guten und waschechten Farbergebnissen geführt.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein erfindungsgemäßes Mittel dadurch gekennzeichnet, dass es mindestens ein filmbildendes, hydrophiles Polymer enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copoylmeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes Keratinmaterial, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

Rahmen einer ersten Ausführungsform kann es bevorzugt sein, wenn die Zubereitung (B), (C) und/oder (D), ganz besonders die Zubereitung (D), mindestens ein nichtionisches, filmbildendes, hydrophiles Polymer entalten.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C₁ bis C₄)-Alkylamino-(C₂ bis C₄)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Stylezeo^{®} CC 10 von der Firma ISP verkauft wird.

Ein kationisches erfindungsgemäßes Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-WasserGemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum .

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Geeignete filmbildende, hydrophile Polymere aus der Gruppe der Acrylamde sind beispielsweise Polymere, welche ausgehend von Monomeren der (Methy)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polyemre der Poly(meth)arylamido-C1-C4-alkyl-sulfonsäuren sind vernetzt und zu mindestens 90 % neutralisiert. Diese Poylmere können vernetzt oder auch unvernetzt sein.

Vernetzte und ganz oder teilweise neutraliserte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clamant unter dem Handelsnamen Hostacerin AMPS vertriebene vernetzte Poly-2-acrylamido-2methyl-propanesulphonsäure-Polymer, das teilweise mit Ammoniak neutralisiert ist.

In einer weiteren explizit ganz besonders bevorzugten Ausführungsform ist ein erfindungsgemäßes Verfahren dadurch gekennzeichnet, dass die Zubereitung (B), (C) und/oder (D), ganz besonders die Zubereitung (D), mindestens ein anionisches, filmbildendes, Polymer enthält.

In diesem Zusammenhang konnten die besten Ergebnisse erhalten werden, wenn die Zubereitung (B), (C) und/oder (D), ganz besonders die Zubereitung (D), mindestens ein filmbildendes Polymer enthält, das mindestens eine Struktureinheit der Formel (P-I) und mindestens eine Struktureinheit der Formel (P-II) umfasst 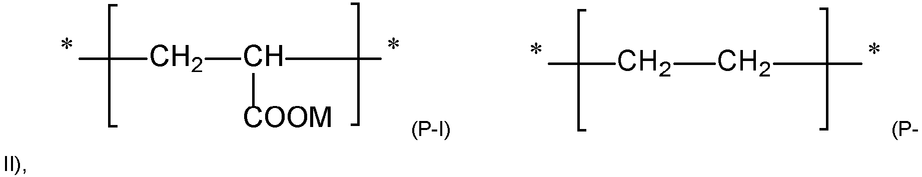 wobei
M für ein Wasserstoffatom oder für Ammonium (NH₄), Natrium, Kalium, ½ Magnesium oder ½ Calcium steht.

In einer weiteren bevorzugten Ausführungsform ist ein erfindungsgemäßes Verfahren dadurch gekennzeichnet, dass die Zubereitung (B), (C) und/oder (D), ganz besonders die Zubereitung (D), mindestens ein filmbildendes Polymer enthält, das mindestens eine Struktureinheit der Formel (P-I) und mindestens eine Struktureinheit der Formel (P-II) umfasst 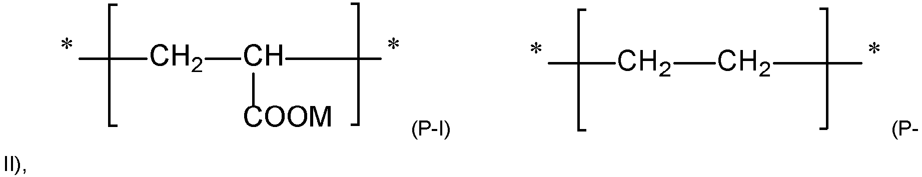 wobei
M für ein Wasserstoffatom oder für Ammonium (NH₄), Natrium, Kalium, ½ Magnesium oder ½ Calcium steht.

Wenn M für ein Wasserstoffatom steht, basiert die Struktureinheit der Formel (P-I) auf einer Acrylsäure-Einheit.

Wenn M für ein Ammonium-Gegenion steht, basiert die Struktureinheit der Formel (P-I) auf dem Ammoniumsalz der Acrylsäure.

Wenn M für ein Natrium-Gegenion steht, basiert die Struktureinheit der Formel (P-I) auf dem Natriumsalz der Acrylsäure.

Wenn M für ein Kalium-Gegenion steht, basiert die Struktureinheit der Formel (P-I) auf dem Kaliumsalz der Acrylsäure.

Wenn M für ein halbes Äquivalent eines Magnesium-Gegenions steht, basiert die Struktureinheit der Formel (P-I) auf dem Magnesiumsalz der Acrylsäure.

Wenn M für ein halbes Äquivalent eines Calcium-Gegenions steht, basiert die Struktureinheit der Formel (P-I) auf dem Calciumsalz der Acrylsäure.

Das oder die erfindungsgemäßen filmbildenden Polymere werden bevorzugt in bestimmten Mengenbereichen im erfindungsgemäßen Zubereitungen (B), (C) und/oder (D) eingesetzt. In diesem Zusammenhang hat es sich zur Lösung der erfindungsgemäßen Aufgabenstellung als besonders bevorzugt erwiesen, wenn die Zubereitung - jeweils bezogen auf ihr Gesamtgewicht- ein oder mehrere filmbildende Polymere in einer Gesamtmenge von 0,1 bis 18,0 Gew.-%, bevorzugt von 1,0 bis 16,0 Gew.-%, weiter bevorzugt von 5,0 bis 14,5 Gew.-% und ganz besonders bevorzugt von 8,0 bis 12,0 Gew.-% enthält.

In einer weiteren bevorzugten Ausführungsform ist ein erfindungsgemäßes Verfahren dadurch gekennzeichnet, dass die Zubereitung (B), (C) und/oder (D) - bezogen auf ihr jewiliges Gesamtgewicht- ein oder mehrere filmbildende Polymere in einer Gesamtmenge von 0,1 bis 18,0 Gew.-%, bevorzugt von 1,0 bis 16,0 Gew.-%, weiter bevorzugt von 5,0 bis 14,5 Gew.-% und ganz besonders bevorzugt von 8,0 bis 12,0 Gew.-% enthält.

### Mehrkomponenten-Verpackungseinheit (Kit-of-parts)

Zur Erhöhung des Anwender-Komforts werden dem Anwender alle für den Anwendungsprozess, insbesondere für den Färbeprozess, notwendigen Zubereitungen in Form einer Mehrkomponenten-Verpackungseinheit (Kit-of-parts) zur Verfügung gestellt.

Ein zweiter Gegenstand der vorliegenden Erfindung ist eine Mehrkomponenten-Verpackungseinheit (Kit-of-parts) zum Behandeln von keratinischem Material, umfassend getrennt voneinander konfektioniert
- einen ersten Container mit einer ersten Zusammensetzung (A) und
- einen zweiten Container mit einer zweiten Zusammensetzung (B), und
- einen dritten Container mit einer Zusammensetzung (C), wobei
wobei die Zusammensetzungen (A), (B) und (C) bereits im Detail bei der Beschreibung des ersten Erfindungsgegenstands offenbart wurden.

Weiterhin kann die erfindungsgemäße Mehrkomponenten-Verpackungseinheit auch noch eine weitere bzw. eine vierte Verpackungseinheit enthaltend eine kosmetische Zubereitung (D) umfassen. Die Zubereitung (D) enthält wie zuvor beschrieben ganz besonders bevorzugt mindestens ein filmbildendes Polymer.

In einer ganz besonders bevorzugten Ausführungsform umfasst die erfindungsgemäße Mehrkomponenten-Verpackungseinheit (Kit-of-parts) getrennt voneinander konfektioniert
- einen weiteren Container mit einer Zusammensetzung (D), wobei die Zusammensetzung (D) bereits im Detail bei der Beschreibung des ersten Erfindungsgegenstands offenbart wurde.

Betreffend die weiteren bevorzugten Ausführungsformen der erfindungsgemäßen Mehrkomponenten-Verpackungseinheit gilt *mutatis mutantis* das zum erfindungsgemäßen Verfahren gesagte.

### Beispiele

### 1. Herstellung des Silan-Blends (Zusammensetzung (A))

Ein Reaktor mit beheizbarer/kühlbarer Außenhülle und mit einem Fassungsvermögen von 10 Litern wurde mit 4,67 kg Methyltrimethoxysilan (34,283 mol) befüllt. Unter Rühren wurden dann 1,33 kg (3-Aminopropyl)triethoxysilan (6,008 mol) hinzugegeben. Dieses Gemisch wurde bei 30 °C gerührt. Im Anschluss daran wurden 670 ml destilliertes Wasser (37,18 mol) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt. Danach wurde ein Vakuum von 280 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 190 Minuten abdestilliert. Im Verlauf der Destillation wurde das Vakuum auf 200 mbar abgesenkt. Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen. Der Wassergehalt betrug kleiner 2,0 Gew.-%.

### 2. Herstellung der Zusammensetzung (B)

Es wurden die folgenden Zusammensetzungen (B) hergestellt (sofern nichts anderes angegeben ist, sind alle Angaben in Gew.-%).

### Zusammensetzung (B)

| | B1 | B2 |
|---|---|---|
| Hydroxyethylcellulose | 1,5 | 1,5 |
| Methylparaben, Natriumsalz | 0,4 | 0,4 |
| Vanillin | --- | 2,5 |
| 1,2-Propandiol | 19,0 | 19,0 |
| Lavanya Zuni (Neelikon Red) CI = 12490 | 0,3 | 0,3 |
| Lavanya Belmont CI = 74160 | 0,1 | 0,1 |
| Lavanya Revolutum (Neelikon Yellow) CI = 11680 | 0,6 | 0,6 |
| TEGO^{®} Solve 90 (Polyglyceryl-6 Caprylate und Polyglyceryl-4 Caprate) | 3,0 | 3,0 |
| Wasser (dest.) | ad 100 | ad 100 |

### 3. Herstellung der Zusammensetzungen (D)

Es wurden die folgenden Zusammensetzungen hergestellt (sofern nichts anderes angegeben ist, sind alle Angaben in Gew.-%).

| Zusammensetzung (D) | Gew.-% |
|---|---|
| Ethylene/Sodium Acrylate Copolymer (25%ige Lösung) | 40,0 |
| Wasser | ad 100 |

### 4. Anwendung

Die anwendungsbereite Zusammensetzung wurde jeweils durch Vermischen von 1,5 g der Zusammensetzung (A) und 20 g der Zusammensetzung (B) hergestellt. Die Zusammensetzungen (A) und (B) wurden jeweils für 1 Minute verschüttelt. Dann wurde dieses anwendungsbereite Mittel jeweils auf zwei Haarsträhnen (Kerling, Euronaturhaar weiß) ausgefärbt.

Eine Minuten nach Beendigung des Verschüttelns wurde die anwendungsbereite Zusammensetzung auf eine erste Strähne (Strähne 1) appliziert, 1 min einwirken gelassen, und danach ausgespült. 25 Minuten nach Beendigung des Verschüttelns wurde die anwendungsbereite Zusammensetzung auf eine zweite Strähne (Strähne 2) appliziert, 1 min einwirken gelassen und danach ausgespült.

Im Anschluss daran wurde die Zusammensetzung (D) auf jede Haarstähne appliziert, für 5 Minuten einwirken gelassen und danach ebenfalls mit Wasser ausgespült.

Die beiden gefärbten Strähnen wurden jeweils getrocknet und visuell unter einer Tageslichtlampe miteinander verglichen.

| Schritt 1: | (A) + B1 | (A) + B2 |
|---|---|---|
| Schritt 2: | (D) | (D) |
| Farbunterschied zwischen Strähne 1 und 2 | hoch | gering |

## Patentansprüche

1. Verfahren zum Behandeln von keratinischem Material, insbesondere menschlichen Haaren, bei dem auf dem keratinischen Material angewendet werden
- eine erste Zusammensetzung (A), die - bezogen auf das Gesamtgewicht der Zusammensetzung (A) - enthält
(A1) weniger als 10 Gew.-% Wasser und
(A21) mindestens ein organisches C₁-C₆-Alkoxy-Silan, das ausgewählt ist aus der Gruppe aus (3-Aminopropyl)triethoxysilan, (3-Aminopropyl)trimethoxysilan, (2-Aminoethyl)triethoxysilan, (2-Aminoethyl)trimethoxysilan, (3-Dimethylaminopropyl)-triethoxysilan, (3-Dimethylaminopropyl)trimethoxysilan, (2-Dimethylaminoethyl)-triethoxysilan, (2-Dimethylaminoethyl)trimethoxysilan und/oder deren Kondensationsprodukten, und
(A22) mindestens ein organisches C₁-C₆-Alkoxysilan, das ausgewählt ist aus der Gruppe aus Methyltrimethoxysilan, Methyltriethoxysilan, Ethyltrimethoxysilan, Ethyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan und/oder deren Kondensationsprodukten, und
- eine zweite Zusammensetzung (B), die enthält
(B1) Wasser und
(B2) einen oder mehrere aromatische oder aliphatische Aldehyde mit 2 bis 20 Kohlenstoffatomen, und
- eine dritte Zusammensetzung (C), die enthält
(C1) mindestens eine farbgebende Verbindung aus der Gruppe der Pigmente und/oder der direktziehenden Farbstoffe.

2. Verfahren nach Anspruch 1, **dadurch gekennzeichnet, dass** die erste Zusammensetzung (A) - bezogen auf das Gesamtgewicht der Zusammensetzung (A) - 0,01 bis 9,5 Gew.-%, bevorzugt 0,01 bis 8,0 Gew.-%, weiter bevorzugt 0,01 bis 6,0 und ganz besonders bevorzugt 0,01 bis 4,0 Gew.-% Wasser (A1) enthält.

3. Verfahren nach einem der Ansprüche 1 bis 2, **dadurch gekennzeichnet, dass** die erste Zusammensetzung (A) - bezogen auf das Gesamtgewicht der Zusammensetzung (A) - ein oder mehrere organische C₁-C₆-Alkoxysilane (A2) und/oder die Kondensationsprodukte hiervon in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

4. Verfahren nach einem der Ansrpüche 1 bis 3, **dadurch gekennzeichnet**, das die erste Zusammensetzung (A) zusätzlich mindestens einen kosmetischen Inhaltsstoff aus der Gruppe aus Hexamethyldisiloxan, Octamethyltrisiloxan, Decamethyltetrasiloxan, Hexamethylcyclotrisiloxan, Octamethylcyclotetrasiloxan und Decamethylcyclopentasiloxan enthält.

5. Verfahren nach einem der Ansprüche 1 bis 4, **dadurch gekennzeichnet, dass** die erste Zusammensetzung (A) - bezogen auf das Gesamtgewicht der Zusammensetzung (A) - 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan enthält.

6. Verfahren nach einem der Ansprüche 1 bis 5, **dadurch gekennzeichnet, dass** die zweite Zuammensetzung (B) - bezogen auf das Gesamtgewicht der Zusammensetzung (B) - 5,0 bis 90,0 Gew.-%, bevorzugt 15,0 bis 85,0 Gew.-%, weiter bevorzugt 25,0 bis 80,0 Gew.-% , nocht weiter bevorzugt 35,0 bis 75,0 Gew.-% und ganz besonders bevorzugt 45,0 bis 70,0 Gew.-% Wasser (B1) enthält.

7. Verfahren nach einem der Ansprüche 1 bis 6, **dadurch gekennzeichnet, dass** die zweite Zusammensetzung (B) mindestens einen aromatischen, carbozyklischen Aldehyd (B2) mit 7 bis 20 Kohlenstoffatomen enthält.

8. Verfahren nach einem der Ansprüche 1 bis 7, **dadurch gekennzeichnet, dass** die zweite Zusammensetzung (B) mindestens einen aromatischen, carbozyklischen Aldehyd (B2) der allgemeinen Formel (A-I) enthält, 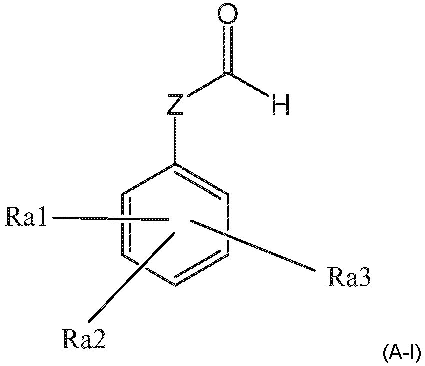 wobei
Ra1, Ra2, Ra3 stehen unabhängig voneinander für ein Wasserstoffatom, eine Hydroxygruppe, eine C₁-C₆-Alkoxygruppe, eine C₁-C₆-Alkylgruppe, ein Halogenatom, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe, oder aber
Ra1 und Ra2 können zusammen mit den Kohlenstoffatomen des Benzenringes, an den sie gebunden sind, einen gesättigten oder ungesättigten, 5-gliedrigen oder 6-gliedrigen heterozyklischen oder carbozyklischen Ring ausbilden, und
Z steht für eine direkte Bindung oder eine Vinylengruppe.

9. Verfahren nach einem der Ansprüche 1 bis 8, **dadurch gekennzeichnet, dass** die zweite Zusammensetzung (B) mindestens einen aromatischen, carbozyklischen Aldehyd (B2) enthält, der ausgewählt ist aus der Gruppe aus 4-Hydroxy-3-methoxybenzaldehyd, 4-Hydroxy-3-ethoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxybenzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 3,5-Dichlor-4-hydroxybenzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd und 4-Diphenylamino-benzaldehyd.

10. Verfahren nach einem der Ansprüche 1 bis 9, **dadurch gekennzeichnet, dass** die zweite Zusammensetzung (B) - bezogen auf das Gesamtgewicht der Zusammensetzung (B) - ein oder mehrere aromatische oder aliphatischen Aldehyde mit 2 bis 20 Kohlenstoffatomen (B2) in einer Gesamtmenge von 0,1 bis 50,0 Gew.-%, bevorzugt 0,5 bis 10,0 Gew.-%, weiter bevorzugt von 0,7 bis 7,0 Gew.-% und ganz besonders bevorzugt von 1,0 bis 4,0 Gew.-% enthält.

11. Verfahren nach einem der Ansprüche 1 bis 10, **dadurch gekennzeichnet, dass** die zweite Zusammensetzung (B) - bezogen auf das Gesamtgewicht der Zusammensetzung (B) - 0,1 bis 50,0 Gew.-%, bevorzugt 0,5 bis 10,0 Gew.-%, weiter bevorzugt von 0,7 bis 7,0 Gew.-% und ganz besonders bevorzugt von 1,0 bis 4,0 Gew.-% Vanillin (B2) enthält.

12. Verfahren nach einem der Ansprüche 1 bis 11, **dadurch gekennzeichnet, dass** die zweite Zusammensetzung (B) zusätzlich einen oder mehrere Fettbestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe enthält.

13. Verfahren nach einem der Ansprüche 1 bis 12, **dadurch gekennzeichnet, dass** die zweite Zusammensetzung (B) ein oder mehrere C₁₂-C₃₀-Fettalkohole aus der Gruppe aus Dodecan-1-ol, Tetradecan-1-ol, Hexadecan-1-ol, Octadecan-1-ol, Eicosan-1-ol, Heneicosan-1-ol, Docosan-1-ol, (9*Z*)-Octadec-9-en-1-ol, (9*E*)-Octadec-9-en-1-ol, (9*Z*,12*Z*)-Octadeca-9,12-dien-1-ol, (9*Z*,12*Z*,15*Z*)-Octadeca-9,12,15-trien-1-ol, (9*Z*)-Eicos-9-en-1-ol, (5*Z*,8*Z*,11*Z*,14*Z*)-Eicosa-5,8,11,14-tetraen-1-ol, (13*Z*)-Docos-13-en-1-ol), (13*E*)-Docosen-1-ol), 2-Octyl-dodecanol, 2-Hexyl-Dodecanol und/oder 2-Butyl-dodecanol enthält.

14. Verfahren nach einem der Ansprüche 1 bis 13, **dadurch gekennzeichnet, dass** die zweite Zusammensetzung (B) mindestens ein C₁₂-C₃₀-Fettsäuremonoglycerid enthielt, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

15. Verfahren nach einem der Ansprüche 1 bis 14, **dadurch gekennzeichnet, dass** die zweite Zusammensetzung (B) mindestens einen Kohlenwasserstoff enthält.

16. Verfahren nach einem der Ansprüche 1 bis 15, **dadurch gekennzeichnet, dass** die zweite Zusammensetzung (B) mindestens ein nichtionisches Tensid enthält.

17. Verfahren nach einem der Ansprüche 1 bis 16, **dadurch gekennzeichnet, dass** die zweite Zusammensetzung (B) mindestens ein verdickendes Polymer, bevorzugt mindestens einen Celluloseether ausgewählt aus der Gruppe aus Hydroxyethylcellulose, Hydroxypropylcellulose und Methylhydroxypropylcellulose, enthält.

18. Verfahren nach einem der Ansprüche 1 bis 17, **dadurch gekennzeichnet, dass** auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde.

19. Verfahren nach einem der Ansprüche 1 bis 18, **dadurch gekennzeichnet, dass** auf dem keratinischen Material eine Zusammensetzung angewendet wird, die unmittelbar vor der Anwendung durch Vermischen der ersten Zusammensetzung (A) mit der zweiten Zusammensetzung (B) und einer dritten Zusammensetzung (C) erhalten wurde.

20. Verfahren nach einem der Ansprüche 1 bis 18, **dadurch gekennzeichnet, dass** in einem ersten Schritt auf dem keratinischen Material eine Zusammensetzung angewendet wird, die unmittelbar vor der Anwendung durch Vermischen der ersten Zusammensetzung (A) und der zweiten Zusammensetzung (B) hergestellt wurde, und in einem zweiten Schritt auf dem keratinischen Material die dritte Zusammensetzung (C) angewendet wird.

21. Verfahren nach einem der Ansprüche 1 bis 20, bei dem auf dem keratinischen Material angewendet wird
- eine weitere Zusammensetzung (D), die enthält mindestens eine filmbildendes Polymer.

22. Verfahren nach einem der Ansprüche 1 bis 21, **dadurch gekennzeichnet, dass** die Zusammensetzung (C) mindestens eine farbgebende Verbindung aus der Gruppe der anorganischen Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

23. Verfahren nach einem der Ansprüche 1 bis 22, **dadurch gekennzeichnet, dass** die Zusammensetzung (C) mindestens eine farbgebende Verbindung aus der Gruppe der organischen Pigmente enthält, die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

24. Verfahren nach einem der Ansprüche 1 bis 23, **dadurch gekennzeichnet, dass** die Zusammensetzung (C) mindestens eine farbgebende Verbindung aus der Gruppe der anionischen, nichtionischen, und/oder kationischen direktziehenden Farbstoffe enthält.

25. Mehrkomponenten-Verpackungseinheit (Kit-of-parts) zum Behandeln von keratinischem Material, umfassend getrennt voneinander konfektioniert
- einen ersten Container mit einer ersten Zusammensetzung (A) und
- einen zweiten Container mit einer zweiten Zusammensetzung (B), und
- einen dritten Container mit einer Zusammensetzung (C),
wobei
die Zusammensetzungen (A) und (B) und (C) in einem der Ansprüche 1 bis 24 definiert sind.

26. Mehrkomponenten-Verpackungseinheit (Kit-of-parts) nach Anspruch 25, umfassend getrennt voneinander konfektioniert
- einen weiteren Container mit einer Zusammensetzung (D), wobei die Zusammensetzung (D) mindestens ein filmbildendes Polymer enthält.

## Claims

1. Method for treating keratinous material, in particular human hair, in which the following are applied to the keratinous material
- a first composition (A) which contains, based on the total weight of composition (A)
(A1) less than 10% by weight of water and
(A21) at least one organic C₁-C₆alkoxy silane selected from the group consisting of (3-aminopropyl)triethoxysilane, (3-aminopropyl)trimethoxysilane, (2-aminoethyl)tri ethoxysilane, (2-aminoethyl)trimethoxysilane, (3-dimethylaminopropyl)triethoxysilane, (3-dimethylaminopropyl)trimethoxysilane, (2-dimethylaminoethyl)triethoxysilane, (2-dimethylaminoethyl)trimethoxysilane and/or their condensation products, and
(A22) at least one organic C₁-C₆alkoxysilane selected from the group consisting of methyltrimethoxysilane, methyltriethoxysilane, ethyltrimethoxysilane, ethyltriethoxysilane, hexyltrimethoxysilane, hexyltriethoxysilane, octyltrimethoxysilane, octyltriethoxysilane, dodecyltrimethoxysilane, dodecyltriethoxysilane, and/or their condensation products, and
- a second composition (B) containing
(B1) water and
(B2) one or more aromatic or aliphatic aldehydes having 2 to 20 carbon atoms, and
- a third composition (C) containing
(C1) at least one coloring compound from the group of pigments and/or direct dyes.

2. Method according to claim 1, **characterized in that** the first composition (A) - based on the total weight of the composition (A) - contains 0.01 to 9.5 wt.%, preferably 0.01 to 8.0 wt.%, more preferably 0.01 to 6.0 wt.%, and most preferably 0.01 to 4.0 wt.% water (A1).

3. Method according to one of claims 1 to 2, **characterized in that** the first composition (A) - based on the total weight of composition (A) - contains one or more organic C₁ -C₆ -alkoxysilanes (A2) and/or the condensation products thereof in a total amount of 30.0 to 85.0 wt.%, preferably 35.0 to 80.0 wt.%, more preferably from 40.0 to 75.0 wt.%, even more preferably from 45.0 to 70.0 wt.% and most preferably from 50.0 to 65.0 wt.%.

4. Method according to one of claims 1 to 3, **characterized in that** the first composition (A) additionally contains at least one cosmetic ingredient from the group consisting of hexamethyldisiloxane, octamethyltrisiloxane, decamethyltetrasiloxane, hexamethylcyclotrisiloxane, octamethylcyclotetrasiloxane, and decamethylcyclopentasiloxane.

5. Method according to one of claims 1 to 4, **characterized in that** the first composition (A) contains - based on the total weight of composition (A) - contains 10.0 to 50.0 wt.%, preferably 15.0 to 45.0 wt.%, more preferably 20.0 to 40.0 wt.%, even more preferably 25.0 to 35.0 wt.%, and most preferably 31.0 to 34.0 wt.% hexamethyldisiloxane.

6. Method according to one of claims 1 to 5, **characterized in that** the second composition (B) contains - based on the total weight of composition (B) -
5.0 to 90.0 wt.%, preferably 15.0 to 85.0 wt.%, more preferably 25.0 to 80.0 wt.%, even more preferably 35.0 to 75.0 wt.%, and most preferably 45.0 to 70.0 wt.% water (B1).

7. Method according to one of claims 1 to 6, **characterized in that** the second composition (B) contains at least one aromatic, carbocyclic aldehyde (B2) with 7 to 20 carbon atoms.

8. Method according to one of claims 1 to 7, **characterized in that** the second composition (B) contains at least one aromatic carbocyclic aldehyde (B2) of the general formula (A-I), 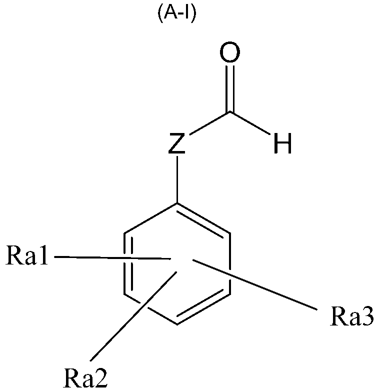 wherein
Ra1, Ra2, Ra3 independently of one another represent a hydrogen atom, a hydroxy group, a C₁-C₆alkoxy group, a C₁-C₆alkyl group, a halogen atom, a C₁-C₆dialkylamino group, a di(C₂-C₆-hydroxyalkyl)amino group, a di(C₁-C₆-alkoxy-C₁-C₆-alkyl)amino group, a C₁-C₆-hydroxyalkyloxy group, a sulfonyl group, a carboxyl group, a sulfonic acid group, a sulfonamido group, a sulfonamide group, a carbamoyl group, a C₂-C₆- acyl group, an acetyl group, or a nitro group, or
Ra1 and Ra2 together with the carbon atoms of the benzene ring to which they are bound, may form a saturated or unsaturated, 5-membered or 6-membered heterocyclic or carbocyclic ring, and
Z represents a direct bond or a vinyl group.

9. Method according to one of claims 1 to 8, **characterized in that** the second composition (B) contains at least one aromatic, carbocyclic aldehyde (B2) selected from the group consisting of 4-hydroxy-3-methoxybenzaldehyde, 4-hydroxy-3-ethoxybenzaldehyde, 3,5-dimethoxy-4-hydroxybenzaldehyde, 4-hydroxy-1-naphthaldehyde, 4-hydroxy-2-methoxybenzaldehyde, 3,4-dihydroxy-5-methoxybenzaldehyde, 3,4,5-trihydroxybenzaldehyde, 3,5-dibromo-4-hydroxybenzaldehyde, 4-hydroxy-3-nitrobenzaldehyde, 3-bromo-4-hydroxybenzaldehyde, 4-hydroxy-3-methylbenzaldehyde, 3,5-dimethyl-4-hydroxybenzaldehyde, 5-bromo-4-hydroxy-3-methoxybenzaldehyde, 4-diethylamino-2-hydroxybenzaldehyde, 4-dimethylamino-2-methoxybenzaldehyde, coniferyl aldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxybenzaldehyde, 2,4-dihydroxy-3-methylbenzaldehyde, 2,4-dihydroxy-5-methylbenzaldehyde, 2,4-dihydroxy-6-methylbenzaldehyde, 2,4-dihydroxy-3-methoxybenzaldehyde, 2,4-dihydroxy-5-methoxybenzaldehyde, 2,4-dihydroxy-6-methoxybenzaldehyde, 2,5-dihydroxybenzaldehyde, 2,6-dihydroxybenzaldehyde, 3,4-dihydroxybenzaldehyde, 3,4-dihydroxy-2-methylbenzaldehyde, 3,4-dihydroxy-5-methylbenzaldehyde, 3,4-dihydroxy-6-methylbenzaldehyde, 3,4-dihydroxy-2-methoxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 4-dimethylaminobenzaldehyde, 4-diethylaminobenzaldehyde, 4- dimethylamino-2-hydroxybenzaldehyde, 3,5-dichloro-4-hydroxybenzaldehyde, 3-chloro-4-hydroxybenzaldehyde, 5-chloro-3,4-dihydroxybenzaldehyde, 5-bromo-3,4-dihydroxybenzaldehyde, 3-Chloro-4-hydroxy-5-methoxybenzaldehyde, 2-Methoxy-1-naphthaldehyde, 4-Methoxy-1-naphthaldehyde, 2-Hydroxy-1-naphthaldehyde, 2,4-Dihydroxy-1-naphthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-dimethylamino-1-naphthaldehyde, 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 5-nitrovanillin, 2,5-dinitrosalicylaldehyde, 5-bromo-3-nitrosalicylaldehyde, 4-nitro-1-naphthaldehyde, 2-nitrozimtaldehyde, 3-nitrozimtaldehyde, 4-nitrozimtaldehyde, 4-dimethylaminozimtaldehyde, 2-dimethylaminobenzaldehyde, 2-chloro-4-dimethylaminobenzaldehyde, 4-dimethylamino-2-methylbenzaldehyde, 4-diethylamino-cinnamaldehyde, 4-dibutylamino-benzaldehyde, and 4-diphenylamino-benzaldehyde.

10. Method according to one of claims 1 to 9, **characterized in that** the second composition (B) contains, based on the total weight of composition (B), one or more aromatic or aliphatic aldehydes with 2 to 20 carbon atoms (B2) in a total amount of 0.1 to 50.0 wt.%, preferably 0.5 to 10.0 wt.%, more preferably from 0.7 to 7.0% by weight and most preferably from 1.0 to 4.0% by weight.

11. Method according to one of claims 1 to 10, **characterized in that** the second composition (B) contains - based on the total weight of the composition (B) - contains 0.1 to 50.0 wt.%, preferably 0.5 to 10.0 wt.%, more preferably 0.7 to 7.0 wt.% and most preferably 1.0 to 4.0 wt.% vanillin (B2).

12. Method according to one of claims 1 to 11, **characterized in that** the second composition (B) additionally contains one or more fat components from the group of C₁₂ -C₃₀ -fatty alcohols, C₁₂ - C₃₀ -fatty acid triglycerides, C₁₂ -C₃₀ fatty acid monoglycerides, C₁₂ -C₃₀ fatty acid diglycerides and/or hydrocarbons.

13. Method according to one of claims 1 to 12, **characterized in that** the second composition (B) contains one or more C₁₂ -C₃₀fatty alcohols from the group consisting of dodecan-1-ol, tetradecan-1-ol, hexadecan-1-ol, octadecan-1-ol, eicosan-1-ol, heneicosan-1-ol, docosan-1-ol, (9Z)-octadec-9-en-1-ol, (9E)-Octadec-9-en-1-ol, (9Z,12Z)-Octadeca-9,12-dien-1-ol, (9Z,12Z,15Z)-Octadeca-9,12,15-trien-1-ol, (9Z)-Eicos-9-en-1-ol, (5Z,8Z,11Z,14Z)-Eicosa-5,8,11,14-tetraen-1-ol, (13Z)-Docos-13-en-1-ol), (13E)-Docosen-1-ol), 2-Octyl-dodecanol, 2-hexyl-dodecanol and/or 2-butyl-dodecanol.

14. Method according to one of claims 1 to 13, **characterized in that** the second composition (B) contains at least one C₁₂ -C₃₀ fatty acid monoglyceride selected from the monoesters of glycerol with one equivalent of fatty acid from the group consisting of dodecanoic acid, tetradecanoic acid, hexadecanoic acid, tetracosanoic acid, octadecanoic acid, eicosanoic acid, and/or docosanoic acid.

15. Method according to any one of claims 1 to 14, **characterized in that** the second composition (B) contains at least one hydrocarbon.

16. Method according to one of claims 1 to 15, **characterized in that** the second composition (B) contains at least one nonionic surfactant.

17. Method according to one of claims 1 to 16, **characterized in that** the second composition (B) contains at least one thickening polymer, preferably at least one cellulose ether selected from the group consisting of hydroxyethyl cellulose, hydroxypropyl cellulose, and methylhydroxypropyl cellulose.

18. Method according to one of claims 1 to 17, **characterized in that** a composition prepared immediately prior to application by mixing the first composition (A) and the second composition (B) is applied to the keratinous material.

19. Method according to one of claims 1 to 18, **characterized in that** a composition obtained immediately prior to application by mixing the first composition (A) with the second composition (B) and a third composition (C) is applied to the keratinous material.

20. Method according to one of claims 1 to 18, **characterized in that**, in a first step, a composition is applied to the keratinous material, which was prepared immediately before application by mixing the first composition (A) and the second composition (B), and in a second step, the third composition (C) is applied to the keratinous material.

21. Method according to one of claims 1 to 20, wherein the following is applied to the keratinous material
- a further composition (D) containing at least one film-forming polymer.

22. Method according to one of claims 1 to 21, **characterized in that** composition (C) contains at least one coloring compound from the group of inorganic pigments selected from the group of colored metal oxides, metal hydroxides, metal oxide hydrates, silicates, metal sulfides, complex metal cyanides, metal sulfates, bronze pigments, and/or colored pigments based on mica or glimmer, which are coated with at least one metal oxide and/or one metal oxychloride.

23. Method according to one of claims 1 to 22, **characterized in that** composition (C) contains at least one coloring compound from the group of organic pigments selected from the group consisting of carmine, quinacridone, phthalocyanine, sorghum, blue pigments with Color Index numbers CI 42090, CI 69800, CI 69825, CI 73000, CI 74100, CI 74160, yellow pigments with the Color Index numbers CI 11680, CI 11710, CI 15985, CI 19140, CI 20040, CI 21100, CI 21108, CI 47000, CI 47005, green pigments with Color Index numbers CI 61565, CI 61570, CI 74260, orange pigments with Color Index numbers CI 11725, CI 15510, CI 45370, CI 71105, red pigments with Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, and/or CI 75470.

24. Method according to one of claims 1 to 23, **characterized in that** composition (C) contains at least one coloring compound from the group of anionic, nonionic, and/or cationic direct dyes.

25. Multi-component packaging unit (kit of parts) for treating keratinous material, comprising separately packaged
- a first container with a first composition (A) and
- a second container with a second composition (B), and
- a third container with a composition (C),
wherein
the compositions (A) and (B) and (C) are defined in one of claims 1 to 24.

26. Multi-component packaging unit (kit of parts) according to claim 25, comprising separately packaged
- a further container with a composition (D), wherein the composition (D) contains at least one film-forming polymer.

## Revendications

1. Procédé de traitement de matière kératinique, en particulier de cheveux humains, dans lequel on applique sur la matière kératinique
- une première composition (A) qui contient, par rapport au poids total de la composition (A)
(A1) moins de 10 % en poids d'eau et
(A21) au moins un silane organique en C₁ -C₆ -alcoxy, choisi dans le groupe constitué par le (3-aminopropyl)triéthoxysilane, le (3-aminopropyl)triméthoxysilane, le (2-aminoéthyl)triéthoxysilane, (2-aminoéthyl)triméthoxysilane, (3-diméthylamino-propyl)triéthoxysilane, (3-diméthylaminopropyl)triméthoxysilane, (2-diméthyl-aminoéthyl)triéthoxysilane, (2-diméthylaminoéthyl)triméthoxysilane et/ou leurs produits de condensation, et
(A22) au moins un alcoxysilane organique en C₁à C₆choisi dans le groupe constitué par le méthyltriméthoxysilane, le méthyltriéthoxysilane, l'éthyltriméthoxysilane, l'éthyltriéthoxysilane, l'hexyltriméthoxysilane, l'hexyltriéthoxysilane, l'octyltriméthoxysilane, l'octyltriéthoxysilane, le dodécyltriméthoxysilane, le dodécyltriéthoxysilane et/ou leurs produits de condensation, et
- une deuxième composition (B) qui contient
(B1) de l'eau et
(B2) un ou plusieurs aldéhydes aromatiques ou aliphatiques ayant de 2 à 20 atomes de carbone, et
- une troisième composition (C) qui contient
(C1) au moins un composé colorant choisi dans le groupe des pigments et/ou des colorants directs.

2. Procédé selon la revendication 1, **caractérisé en ce que** la première composition (A) contient, par rapport au poids total de la composition (A) - 0,01 à 9,5 % en poids, de préférence 0,01 à 8,0 % en poids, de préférence encore 0,01 à 6,0 % en poids et de manière particulièrement préférée 0,01 à 4,0 % en poids d'eau (A1).

3. Procédé selon l'une des revendications 1 à 2, **caractérisé en ce que** la première composition (A) contient, par rapport au poids total de la composition (A), un ou plusieurs C₁ -C₆ -alcoxysilanes (A2) et/ou leurs produits de condensation en une quantité totale de 30,0 à 85,0 % en poids, de préférence de 35,0 à 80,0 % en poids, de préférence d' 40,0 à 75,0 % en poids, encore plus préférentiellement de 45,0 à 70,0 % en poids et tout particulièrement de 50,0 à 65,0 % en poids.

4. Procédé selon l'une des revendications 1 à 3, **caractérisé en ce que** la première composition (A) contient en outre au moins un ingrédient cosmétique choisi dans le groupe constitué par l'hexaméthyldisiloxane, l'octaméthyltrisiloxane, décaméthyltétrasiloxane, hexaméthylcyclotrisiloxane, octaméthylcyclotétrasiloxane et décaméthylcyclopentasiloxane.

5. Procédé selon l'une des revendications 1 à 4, **caractérisé en ce que** la première composition (A) contient, par rapport au poids total de la composition (A) - contient 10,0 à 50,0 % en poids, de préférence 15,0 à 45,0 % en poids, de préférence encore 20,0 à 40,0 % en poids, de préférence encore plus 25,0 à 35,0 % en poids et de manière tout à fait préférée 31,0 à 34,0 % en poids d'hexaméthyldisiloxane.

6. Procédé selon l'une des revendications 1 à 5, **caractérisé en ce que** la deuxième composition (B) contient, par rapport au poids total de la composition (B),
contient 5,0 à 90,0 % en poids, de préférence 15,0 à 85,0 % en poids, de manière encore plus préférée 25,0 à 80,0 % en poids , de manière encore plus préférée 35,0 à 75,0 % en poids et de manière particulièrement préférée 45,0 à 70,0 % en poids d'eau (B1).

7. Procédé selon l'une des revendications 1 à 6, **caractérisé en ce que** la deuxième composition (B) contient au moins un aldéhyde carbocyclique aromatique (B2) comportant 7 à 20 atomes de carbone.

8. Procédé selon l'une des revendications 1 à 7, **caractérisé en ce que** la deuxième composition (B) contient au moins un aldéhyde carbocyclique aromatique (B2) de formule générale (A-I), 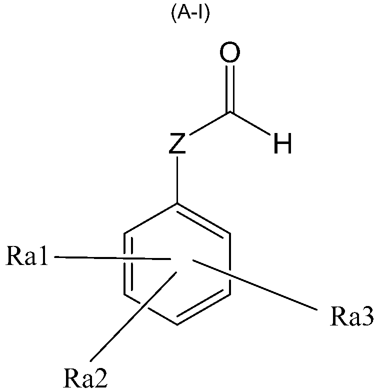 dans laquelle
Ra1, Ra2, Ra3 représentent indépendamment l'un de l'autre un atome d'hydrogène, un groupe hydroxy, un groupe alcoxy ₑₙ C₁ -C₆, un groupe alkyle ₑₙ C₁ -C(₆), un atome d'halogène, un groupe dialkylamino ₑₙ C₁ -C₆, un groupe di(C₂ -C₆ - hydroxyalkyl)amino, un groupe di(C₁ -C₆ -alcoxy-C₁ -C₆ -alkyl)amino, un groupe C₁-C₆-hydroxyalkyloxy, un groupe sulfonyle, un groupe carboxyle, un groupe acide sulfonique, un groupe sulfonamido, un groupe sulfonamide, un groupe carbamoyle, un groupe acyle en C₂-C₆- , un groupe acétyle ou un groupe nitro, ou encore
Ra1 et Ra2 peuvent former, conjointement avec les atomes de carbone du cycle benzénique auxquels ils sont liés, un cycle hétérocyclique ou carbocyclique saturé ou insaturé à 5 ou 6 chaînons, et
Z représente une liaison directe ou un groupe vinylène.

9. Procédé selon l'une des revendications 1 à 8, **caractérisé en ce que** la deuxième composition (B) contient au moins un aldéhyde carbocyclique aromatique (B2) choisi dans le groupe constitué par le 4-hydroxy-3-méthoxybenzaldéhyde, le 4-hydroxy-3-éthoxybenzaldéhyde, 3,5-diméthoxy-4-hydroxybenzaldéhyde, 4-hydroxy-1-naphtaldéhyde, 4-hydroxy-2-méthoxybenzaldéhyde, 3,4-dihydroxy-5-méthoxybenzaldéhyde, 3,4,5-trihydroxybenzaldéhyde, 3,5-dibromo-4-hydroxybenzaldéhyde, 4-hydroxy-3-nitrobenzaldéhyde, 3-bromo-4-hydroxybenzaldéhyde, 4-hydroxy-3-méthylbenzaldéhyde, 3,5-diméthyl-4-hydroxybenzaldéhyde, 5-bromo-4-hydroxy-3-méthoxybenzaldéhyde, 4-diéthylamino-2-hydroxybenzaldéhyde, 4-diméthylamino-2-méthoxybenzaldéhyde, coniférylaldéhyde, 2-méthoxybenzaldéhyde, 3-méthoxybenzaldéhyde, 4-méthoxybenzaldéhyde, 2-éthoxybenzaldéhyde, 3-éthoxybenzaldéhyde, 4-éthoxybenzaldéhyde, 4-hydroxy-2,3-diméthoxybenzaldéhyde, 4-hydroxy-2,5-diméthoxybenzaldéhyde, 4-hydroxy-2,6-diméthoxybenzaldéhyde, 4-hydroxy-2-méthylbenzaldéhyde, 4-hydroxy-2,3-diméthyl-benzaldéhyde, 4-hydroxy-2,5-diméthylbenzaldéhyde, 4-hydroxy-2,6-diméthylbenzaldéhyde, 3,5-diéthoxy-4-hydroxybenzaldéhyde, 2,6-diéthoxy-4-hydroxybenzaldéhyde, 3-hydroxy-4-méthoxybenzaldéhyde, 2-hydroxy-4-méthoxy-benzaldéhyde, 2-éthoxy-4-hydroxy-benzaldéhyde, 3-éthoxy-4-hydroxy-benzaldéhyde, 4-éthoxy-2-hydroxy-benzaldéhyde, 4-éthoxy-3-hydroxybenzaldéhyde, 2,3-diméthoxybenzaldéhyde, 2,4-diméthoxybenzaldéhyde, 2,5-diméthoxybenzaldéhyde, 2,6-diméthoxybenzaldéhyde, 3,4-diméthoxybenzaldéhyde, 3,5-diméthoxybenzaldéhyde, 2,3,4-triméthoxybenzaldéhyde, 2,3,5-triméthoxybenzaldéhyde, 2,3,6-triméthoxybenzaldéhyde, 2,4,6-triméthoxybenzaldéhyde, 2,4,5-triméthoxybenzaldéhyde, 2,5,6-triméthoxybenzaldéhyde, 2-hydroxybenzaldéhyde, 3-hydroxybenzaldéhyde, 4-hydroxybenzaldéhyde, 2,3-dihydroxybenzaldéhyde, 2,4-dihydroxybenzaldéhyde, 2,4-dihydroxy-3-méthyl-benzaldéhyde, 2,4-dihydroxy-5-méthyl-benzaldéhyde, 2,4-dihydroxy-6-méthyl-benzaldéhyde, 2,4-dihydroxy-3-méthoxy-benzaldéhyde, 2,4-dihydroxy-5-méthoxy-benzaldéhyde, 2,4-dihydroxy-6-méthoxy-benzaldéhyde, 2,5-dihydroxybenzaldéhyde, 2,6-dihydroxybenzaldéhyde, 3,4-dihydroxybenzaldéhyde, 3,4-dihydroxy-2-méthyl-benzaldéhyde, 3,4-dihydroxy-5-méthyl-benzaldéhyde, 3,4-dihydroxy-6-méthyl-benzaldéhyde, 3,4-dihydroxy-2-méthoxy-benzaldéhyde, 3,5-dihydroxybenzaldéhyde, 2,3,4-trihydroxybenzaldéhyde, 2,3,5-trihydroxybenzaldéhyde, 2,3,6-trihydroxybenzaldéhyde, 2,4,6-trihydroxybenzaldéhyde, 2,4,5-trihydroxybenzaldéhyde, 2,5,6-trihydroxybenzaldéhyde, 4-diméthylaminobenzaldéhyde, 4-diéthylaminobenzaldéhyde, 4- diméthylamino-2-hydroxybenzaldéhyde, 3,5-dichloro-4-hydroxybenzaldéhyde, 3-chloro-4-hydroxybenzaldéhyde, 5-chloro-3,4-dihydroxybenzaldéhyde, 5-bromo-3,4-dihydroxybenzaldéhyde, 3-chloro-4-hydroxy-5-méthoxybenzaldéhyde, 2-méthoxy-1-naphtaldéhyde, 4-méthoxy-1-naphtaldéhyde, 2-hydroxy-1-naphtaldéhyde, 2,4-dihydroxy-1-naphtaldéhyde, 4-hydroxy-3-méthoxy-1-naphtaldéhyde, 2-hydroxy-4-méthoxy-1-naphtaldéhyde, 3-hydroxy-4-méthoxy-1-naphtaldéhyde, 2,4-diméthoxy-1-naphtaldéhyde, 3,4-diméthoxy-1-naphtaldéhyde, 4-diméthylamino-1-naphtaldéhyde, 2-nitrobenzaldéhyde, 3-nitrobenzaldéhyde, 4-nitrobenzaldéhyde, 4-méthyl-3-nitrobenzaldéhyde, 3-hydroxy-4-nitrobenzaldéhyde, 5-hydroxy-2-nitrobenzaldéhyde, 2-hydroxy-5-nitrobenzaldéhyde, 2-hydroxy-3-nitrobenzaldéhyde, 2-fluoro-3-nitrobenzaldéhyde, 3-méthoxy-2-nitrobenzaldéhyde, 4-chloro-3-nitrobenzaldéhyde, 2-chloro-6-nitrobenzaldéhyde, 5-chloro-2-nitrobenzaldéhyde, 4-chloro-2-nitrobenzaldéhyde, 2,4-dinitrobenzaldéhyde, 2,6-dinitrobenzaldéhyde, 2-hydroxy-3-méthoxy-5-nitrobenzaldéhyde, 4,5-diméthoxy-2-nitrobenzaldéhyde, 5-nitrovanilline, 2,5-dinitrosalicylaldéhyde, 5-bromo-3-nitrosalicylaldéhyde, 4-nitro-1-naphtaldéhyde, 2-nitro-cinnamaldéhyde, 3-nitro-cinnamaldéhyde, 4-nitro-cinnamaldéhyde, 4-diméthylamino-cinnamaldéhyde, 2-diméthylaminobenzaldéhyde, 2-chloro-4-diméthylaminobenzaldéhyde, 4-diméthylamino-2-méthylbenzaldehyde, 4-diéthylamino-cinnamaldéhyde, 4-dibutylamino-benzaldehyde et 4-diphénylamino-benzaldehyde.

10. Procédé selon l'une des revendications 1 à 9, **caractérisé en ce que** la deuxième composition (B) contient, par rapport au poids total de la composition (B), un ou plusieurs aldéhydes aromatiques ou aliphatiques ayant de 2 à 20 atomes de carbone (B2) en une quantité totale de 0,1 à 50,0 % en poids, de préférence de 0,5 à 10,0 % en poids, de préférence de 0,7 à 7,0 % en poids et de manière particulièrement préférée de 1,0 à 4,0 % en poids.

11. Procédé selon l'une des revendications 1 à 10, **caractérisé en ce que** la deuxième composition (B) contient, par rapport au poids total de la composition (B) - contient 0,1 à 50,0 % en poids, de préférence 0,5 à 10,0 % en poids, de manière encore plus préférée 0,7 à 7,0 % en poids et de manière tout à fait particulièrement préférée 1,0 à 4,0 % en poids de vanilline (B2).

12. Procédé selon l'une des revendications 1 à 11, **caractérisé en ce que** la deuxième composition (B) contient en outre un ou plusieurs composants gras choisis dans le groupe des alcools gras ₑₙ C_{12à} C₃₀, des triglycérides d'acides gras ₑₙ C₁₂ à C₃₀, des₁₂ -C₃₀ , des diglycérides d'acides gras en C₁₂ -C₃₀ et/ou des hydrocarbures.

13. Procédé selon l'une des revendications 1 à 12, **caractérisé en ce que** la deuxième composition (B) contient un ou plusieurs alcools gras en C₁₂₃₀ du groupe comprenant le dodécan-1-ol, le tétradécan-1-ol, l'hexadécan-1-ol, l'octadécan-1-ol, l'eicosan-1-ol, l'hénéicosan-1-ol, le docosan-1-ol, le (9*Z*)-octadéc-9-én-1-ol, le (9E)-Octadec-9-en-1-ol, (9Z,12Z)-Octadeca-9,12-dien-1-ol, (9Z,12Z,15Z)-Octadeca-9,12,15-trien-1-ol, (9Z)-Eicos-9-en-1-ol, (5Z,8Z,11Z,14Z)-Eicosa-5,8,11,14-tetraen-1-ol, (13Z)-Docos-13-en-1-ol), (13E)-Docosen-1-ol), 2-Octyl-dodecanol, 2-hexyl-dodécanol et/ou 2-butyl-dodécanol.

14. Procédé selon l'une des revendications 1 à 13, **caractérisé en ce que** la deuxième composition (B) contient au moins un monoglycéride d'acide gras en C₁₂₃₀ -acide gras, qui est choisi parmi les monoesters de glycérol avec un équivalent d'acide gras du groupe constitué par l'acide dodécanoïque, l'acide tétradécanoïque, l'acide hexadécanoïque, l'acide tétracosanoïque, l'acide octadécanoïque, l'acide eicosanoïque et/ou l'acide docosanoïque.

15. Procédé selon l'une des revendications 1 à 14, **caractérisé en ce que** la deuxième composition (B) contient au moins un hydrocarbure.

16. Procédé selon l'une des revendications 1 à 15, **caractérisé en ce que** la deuxième composition (B) contient au moins un tensioactif non ionique.

17. Procédé selon l'une des revendications 1 à 16, **caractérisé en ce que** la deuxième composition (B) contient au moins un polymère épaississant, de préférence au moins un éther de cellulose choisi dans le groupe constitué par l'hydroxyéthylcellulose, l'hydroxypropylcellulose et la méthylhydroxypropylcellulose.

18. Procédé selon l'une des revendications 1 à 17, **caractérisé en ce qu'**on applique sur la matière kératinique une composition préparée immédiatement avant l'application par mélange de la première composition (A) et de la deuxième composition (B).

19. Procédé selon l'une des revendications 1 à 18, **caractérisé en ce qu'**une composition obtenue immédiatement avant l'application en mélangeant la première composition (A) avec la deuxième composition (B) et une troisième composition (C) est appliquée sur le matériau kératinique.

20. Procédé selon l'une des revendications 1 à 18, **caractérisé en ce que**, dans une première étape, on applique sur le matériau kératinique une composition préparée immédiatement avant l'application en mélangeant la première composition (A) et la deuxième composition (B) de l' , et dans une deuxième étape, on applique sur le matériau kératinique la troisième composition (C).

21. Procédé selon l'une des revendications 1 à 20, dans lequel on applique sur le matériau kératinique
- une autre composition (D) qui contient au moins un polymère filmogène.

22. Procédé selon l'une des revendications 1 à 21, **caractérisé en ce que** la composition (C) contient au moins un composé colorant du groupe des pigments inorganiques, qui est choisi dans le groupe des oxydes métalliques colorés, des hydroxydes métalliques, des hydrates d'oxydes métalliques, des silicates, sulfures métalliques, cyanures métalliques complexes, sulfates métalliques, pigments de bronze et/ou de pigments colorés à base de mica ou de micacés, qui sont revêtus d'au moins un oxyde métallique et/ou un oxychlorure métallique.

23. Procédé selon l'une des revendications 1 à 22, **caractérisé en ce que** la composition (C) contient au moins un composé colorant choisi dans le groupe des pigments organiques, qui est sélectionné dans le groupe constitué par le carmin, la quinacridone, la phtalocyanine, le sorgho, les pigments bleus ayant les numéros d'index de couleur CI 42090, CI 69800, CI 69825, CI 73000, CI 74100, CI 74160, des pigments jaunes ayant les numéros d'index de couleur CI 11680, CI 11710, CI 15985, CI 19140, CI 20040, CI 21100, CI 21108, CI 47000, CI 47005, pigments verts portant les numéros d'index de couleur CI 61565, CI 61570, CI 74260, pigments orange portant les numéros d'index de couleur CI 11725, CI 15510, CI 45370, CI 71105, pigments rouges avec les numéros d'index de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

24. Procédé selon l'une des revendications 1 à 23, **caractérisé en ce que** la composition (C) contient au moins un composé colorant du groupe des colorants anioniques, non ioniques et/ou cationiques à fixation directe.

25. Unité d'emballage à plusieurs composants (kit de pièces) pour le traitement de matière kératinique, comprenant, conditionnés séparément les uns des autres
- un premier récipient contenant une première composition (A) et
- un deuxième récipient contenant une deuxième composition (B), et
- un troisième conteneur avec une composition (C),
où
les compositions (A) et (B) et (C) sont définies dans l'une des revendications 1 à 24.

26. Unité d'emballage à plusieurs composants (kit de pièces) selon la revendication 25, comprenant des composants emballés séparément les uns des autres
- un autre récipient contenant une composition (D), la composition (D) contenant au moins un polymère filmogène.
